# EUROPEAN PATENT SPECIFICATION

(11) **EP 4 499 038 B1**
(45) Date of publication and mention of the grant of the patent: **03.12.2025**
(21) Application number: 23715487.7
(22) Date of filing: 27.03.2023
(51) Int. Cl.: A61K 9/00, A61K 9/14, A61K 9/20, A61K 31/4375, A61K 31/47, A61P 7/00, A61P 17/00, A61P 1/00

(54) **SOLID COMPOSITION COMPRISING SOLUBILISED BRADYKININ B2-RECEPTOR ANTAGONISTS**
FESTE ZUSAMMENSETZUNG, DIE SOLUBILISIERTE BRADYKININ B2-REZEPTOR-ANTAGONISTEN UMFASST
COMPOSITION SOLIDE COMPRENANT DES ANTAGONISTES SOLUBILISÉS DE RÉCEPTEUR B2 DE LA BRADYKININE

(30) Priority: 25.03.2022 EP 22164462
(43) Date of publication of application: 05.02.2025
(73) Proprietor: Pharvaris GmbH, 6300 Zug (CH)
(72) Inventor: GIBSON, Christoph, 6300 Zug (CH)
(74) Representative: Walter, Kai U.
(86) International application number: PCT/EP2023/057791
(87) International publication number: WO 2023/180575

(56) References cited:
- WO-A1-2019/101906
- WO-A1-2020/234480
- WO-A1-2022/101395
- LESAGE ANNE ET AL: "In Vitro Pharmacological Profile of a New Small Molecule Bradykinin B2 Receptor Antagonist", FRONTIERS IN PHARMACOLOGY, vol. 11, 19 June 2020 (2020-06-19), XP055793032, DOI: 10.3389/fphar.2020.00916
- PAROJCIC JELENA ET AL: "An Investigation into the Factors Influencing Drug Release from Hydrophilic Matrix Tablets Based on Novel Carbomer Polymers", vol. 11, no. 1, 1 January 2004 (2004-01-01), US, pages 59 - 65, XP055954611, ISSN: 1071-7544, Retrieved from the Internet <URL:https://core.ac.uk/download/pdf/237391013.pdf> DOI: 10.1080/10717540490265379
- CONTE U ET AL: "Modulation of the dissolution profiles from Geomatrix@? multi-layer matrix tablets containing drugs of different solubility", BIOMATERIALS, ELSEVIER, AMSTERDAM, NL, vol. 17, no. 9, 1 May 1996 (1996-05-01), pages 889 - 896, XP004032726, ISSN: 0142-9612, DOI: 10.1016/0142-9612(96)83284-4
- TZIVELEKA LETO-AIKATERINI ET AL: "Ulvan, a bioactive marine sulphated polysaccharide as a key constituent of hybrid biomaterials: A review", CARBOHYDRATE POLYMERS, vol. 218, 29 April 2019 (2019-04-29), pages 355 - 370, XP085719591, ISSN: 0144-8617, DOI: 10.1016/J.CARBPOL.2019.04.074
- DWAYNE T. FRIESEN ET AL: "Hydroxypropyl Methylcellulose Acetate Succinate-Based Spray-Dried Dispersions: An Overview", MOLECULAR PHARMACEUTICS, vol. 5, no. 6, 1 December 2008 (2008-12-01), pages 1003 - 1019, XP055056966, ISSN: 1543-8384, DOI: 10.1021/mp8000793

## Description

### BACKGROUND OF THE INVENTION

The present invention relates to a solid dispersion comprising a bradykinin (BK) B2 receptor antagonist having a chemical structure according to Formula (1), to methods of preparing such solid dispersions, and to their uses as medicaments in the treatment of subjects that may benefit from a bradykinin B2 receptor antagonist.

Bradykinin (BK) is a peptide hormone that participates in inflammatory processes by activation of endothelial cells leading to vasodilation, increased vascular permeability, production of nitric oxide, and mobilization of arachidonic acid. BK also stimulates sensory nerve endings causing a burning dysaesthesia. Thus, the classical parameters of inflammation (e.g. redness, heat, swelling and pain) can all result from BK formation. BK is a short-lived component of the kallikrein-kinin system. The concentration of circulating BK is maintained at a low level under normal physiological conditions and may be rapidly increased under pathological situations by the enzymatic degradation of the circulating glycoprotein precursors called kininogens. The two most potent kininogen-metabolising enzymes are the trypsin-like serine proteases plasma kallikrein and tissue kallikrein. The precursors of these enzymes are normally present in all tissues and are ready to be activated by physiological or pathophysiological processes. The BK B2 receptor is constitutively expressed in most cell and tissue types and mediates most of the known effects of BK when this is produced in plasma or tissues. A large number of in vivo studies have shown that agents that blockade the BK B2 receptor provide therapeutic benefits in pathological conditions such as asthma, allergic rhinitis, pancreatitis, osteoarthritis, traumatic brain injury, Alzheimer's disease, and angioedema.

Numerous peptide and non-peptide antagonists of BK B2 receptor have been described in the prior art. Quinoline derivatives having activity as BK B2 receptor antagonists are, for example, disclosed in WO 2014/159637, WO 2010/031589, WO 2008/116620, WO 2006/40004, WO 03/103671, WO 03/87090, WO 00/23439, WO 00/50418, WO 99/64039, WO 97/41104, WO 97/28153, WO 97/07115, WO 96/13485, EP 0 795 547, EP 0 796 848, EP 0 867 432, and EP 1 213 289. However, these compounds showed a number of deficiencies hampering their utility as a drug, including low metabolic stability, low bioavailability, formation of glutathione adducts and bioactivation (toxicity) as disclosed in WO 2014/159637.

More recently, compounds of Formula (1) were proposed as novel, biologically active and well tolerated BK B2 receptor antagonists (see e.g. WO 2019/101906). While these compounds exhibit attractive pharmacological properties, they also show rather challenging physical or physicochemical properties, including very poor solubility in physiological media.

A compound according to Formula (1) is also known from A. Lesage et al., Frontiers in Pharmacology, vol. 11, 19 June 2020, doi: 10.3389/fphar.2020.00916. The publication describes the in-vitro pharmacological profile of the compound, but does not disclose any specific dosage form or formulation incorporating the compound.

Various formulation techniques have been proposed for poorly soluble drug substances, nevertheless it remains challenging to develop suitable formulations for many new drug candidates with difficult physical and pharmacokinetic properties, and still today many development programmes fail. Among the generally known formulation techniques are solid dispersions. D. T. Friesen et al., Molecular Pharmaceutics, vol. 5, no. 6, December 2008, 1003-1019, doi: 10.1021/mp8000793 describes spray-dried dispersions based on hydroxypropyl methylcellulose acetate. These products are used as oral powders for constitution (OPC) formulations or fill materials for gelatin capsules and intended to achieve a supersaturated concentration of drug in the gastrointestinal fluid. The document does not disclose the incorporation of dispersions into tablets, nor does it relate to dosage forms for extended drug release.

There is a need for formulation designs and pharmaceutical compositions that overcome the difficulties resulting from the challenging properties of compounds according to Formula (1) such as to enable effective oral delivery and achieve significant systemic exposure and bioavailability in human subjects. There is also a need for formulations or pharmaceutical compositions incorporating compounds of Formula (1) such as to achieve a reliable extent of oral absorption. A further need is the provision of formulations or pharmaceutical compositions incorporating compounds of Formula (1) that are stable in their performance and that can be manufactured by established pharmaceutical manufacturing technologies.

Compounds of Formula (1) have a very low water solubility. This makes it challenging to develop an oral formulation that would result in a sufficient and reliable absorption rate, sufficient extent of bioavailability and efficacious plasma levels. The pharmacodynamic profile of the BK B2 receptor antagonists of Formula (1) makes these compounds highly attractive for both acute and chronic treatment, and thus there is a need to overcome the challenges including poor aqueous solubility and develop formulations for these drugs that are viable both in the acute and in the chronic therapeutic setting.

An objective of the present invention is to address one or more of the needs described above. A further objective is to overcome the deficiencies, gaps and limitations of the prior art with respect to the oral delivery of bradykinin B2 receptor antagonists, such as compounds having a chemical structure according to Formula (1). Further objectives will become apparent on the basis of the following description, the Examples and the patent claims.

### SUMMARY OF THE INVENTION

In one aspect, the invention relates to solid dispersions for oral administration comprising a bradykinin B2 receptor antagonist having a chemical structure according to Formula (1), or a salt or solvate thereof: 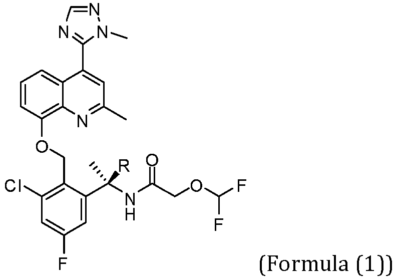 wherein R is deuterium or hydrogen; the solid dispersions are further characterised in that they comprise the BK B2 receptor antagonist in amorphous form, and homogeneously dispersed in a pharmaceutically acceptable polymer. In particular, the BK B2 receptor antagonist may be (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide.

The pharmaceutically acceptable polymer in which the active ingredient is dispersed may, for example, be hydroxypropyl methylcellulose acetate succinate (HPMCAS) or copovidone (PVP/PA).

In a further aspect, the invention relates to pharmaceutical compositions, including extended-release compositions, comprising such solid dispersions. The extended-release compositions may, for example, be in the form of matrix tablets.

In a further aspect, the invention relates to methods of preparing the solid dispersions, such as methods based on melt extrusion or spray drying.

In yet a further aspect, the present invention relates to uses of the solid dispersions or of the extended-release compositions, in particular therapeutic uses. In general, the solid dispersions and the extended-release compositions may be used in the treatment of diseases or conditions responsive to bradykinin B2 receptor modulation. For example, they are advantageous for the treatment of angioedema (AE), including hereditary angioedema (HAE), acquired angioedema (AAE), non-histaminergic idiopathic angioedema, allergic angioedema, drug-induced angioedema as well as angioedema of unidentified cause. The HAE can be of any type, including type I HAE, type II HAE, or type III HAE, preferably type I HAE or type II HAE.

### DESCRIPTION OF THE DRAWINGS

Figure 1 shows mean plasma concentration-time profiles of two different extended-release matrix tablets (formulations 5.1 and 5.2, here referred to as XR1 and XR2) comprising a solid dispersion formulation of a compound according to Formula (1), specifically (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide, here referred to as PHA-022121, compared to an immediate-release capsule formulation of the same drug, in healthy volunteers under fasting conditions.
Figure 2 shows mean plasma concentration-time profiles of an extended-release matrix tablet (formulation 5.1) comprising a solid dispersion formulation of a compound according to Formula (1) in healthy volunteers under fed and fasting conditions.
Figure 3 shows mean plasma concentration-time profiles of an extended-release matrix tablet (formulation 5.2) comprising a solid dispersion formulation of a compound according to Formula (1) in healthy volunteers under fed and fasting conditions.

### DETAILED DESCRIPTION OF THE INVENTION

In a first aspect, the invention provides a solid dispersion for oral administration, comprising (a) a BK B2 receptor antagonist having a chemical structure according to Formula (1), or a salt or solvate thereof: 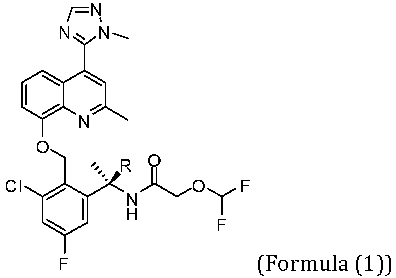 wherein R is deuterium or hydrogen, and (b) at least one pharmaceutically acceptable polymer; wherein the BK B2 receptor antagonist is amorphous and homogeneously dispersed in the polymer.

It has been found by the inventors that the claimed solid dispersions of the BK B2 receptor antagonist of Formula (1) are not only effective in providing rapid and complete release of this pharmacologically active ingredient into physiological media, which is generally useful for designing immediate release compositions that incorporate such solid dispersions, but they are also capable of substantially improving the release of the compound of Formula (1) from extended-release formulations, which has been entirely unexpected. In particular, the solid dispersions may effect a substantially complete release of the active ingredient and reduce the variability of the release rate caused by external factors such as the pH and composition of the liquid medium into which the active compound is released from the extended-release composition, e.g. whether that liquid medium is water, gastric fluid, intestinal fluid, or surrogates thereof, such as simulated gastric fluid, fasted-state simulated intestinal fluid (FaSSIF) or fed-state simulated intestinal fluid (FeSSIF). In contrast, it has been found that conventional extended-release formulation techniques when applied to the compound of Formula (1) result in unreliable drug release, unsatisfactory release profiles, and unpredictable absorption rates.

In this context, a solid dispersion may be understood as a solid material comprising a compound such as a drug substance homogeneously dispersed in a preferably amorphous polymeric matrix. Typically, the dispersed compound is also in the amorphous state, i.e. non-crystalline. For example, the dispersed compound may be molecularly dispersed, or it may be dispersed in the form of non-crystalline domains.

The solid dispersion provided by the inventors comprises the BK B2 receptor antagonist in amorphous form. In a generally preferred embodiment, the amorphous form of the BK B2 receptor antagonist is quantitatively dominant In other words, even if some amount of crystalline BK B2 receptor antagonist is present in the solid dispersion, the quantity of the amorphous form is larger, or even substantially larger, than that of the crystalline form. In another generally preferred embodiment, at least about 90 %, or even at least 95 % or at least 99% of the BK B2 receptor antagonist comprised in the solid dispersion is amorphous. Optionally, all or substantially all of the BK B2 receptor antagonist is in the amorphous form. In this context, the basis of the percentage is the total molar amount of the BK B2 receptor antagonist in the solid dispersion.

In Formula (1), R may be selected from hydrogen and deuterium. In one preferred embodiment, or group of embodiments, R is deuterium, as shown in the formula below:

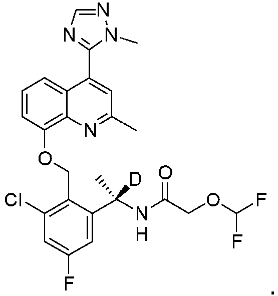

This compound which may also be referred to as (*S*)*-N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide (CAS 2340111-58-0), or alternatively as acetamide, *N*-[(1*S*)-1-[3-chloro-5-fluoro-2-[[[2-methyl-4-(1-methyl-1*H*-1,2,4-triazol-5-yl)-8-quinolinyl]oxy]methyl]phenyl]ethyl-1-d]-2-(difluoromethoxy)-, is a particularly advantageous example of a compound according to Formula (1) in the context of the invention. It should be understood that this preference also applies in combination with all other optional features or preferences disclosed in this description below, whether specifically mentioned or not

Alternatively, in Formula (1), R may be hydrogen, as depicted in the following formula: 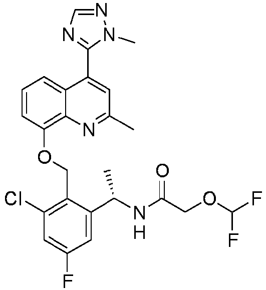 which compound may also be referred to as (*S*)-*N*-(1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide.

The compound of Formula (1) may be present in an essentially non-ionised form, or in ionised form, i.e. in the form of a salt. Moreover, it may optionally be in the form of a solvate. For example, the compound may be a hydrate, such as (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide monohydrate. In one of the generally preferred embodiments, the compound of Formula (1) is non-ionised (i.e. not in salt form) and is defined by R being deuterium.

In a further embodiment, the state of the compound of Formula (1) in the solid dispersion is substantially or even entirely non-ionised. Moreover, the compound of Formula (1) as it is present in the solid dispersion would normally not represent a solvate. However, for the avoidance of doubt, the compound may be in the form of a solvate, when it is combined with the polymer and any other optional constituents to form the solid dispersion as disclosed and claimed herein. For example, a hydrate of the compound, such as the monohydrate, optionally in crystalline form, may be used for preparing the composition. It is believed, however, that upon being incorporated into a solid dispersion, it no longer represents a crystalline material or a hydrate.

In the context of this disclosure, the compound of Formula (1) may also be referred to as (biologically or pharmacologically) active ingredient, drug substance, active pharmaceutical ingredient (API), or the like. Moreover, in one of the further preferred embodiments, the compound of Formula (1) is the sole drug substance contained in the solid dispersion. This is a generally applicable preference within the context of this disclosure.

As mentioned, the solid dispersion comprises at least one pharmaceutically acceptable polymer in which the BK B2 receptor antagonist is dispersed. This polymer may be selected from polymers that are suitable for oral administration and capable of incorporating the BK B2 receptor antagonist in the form of a solid dispersion. In particular, the polymer may be selected from the group consisting of hydrophilic neutral polymers and hydrophilic anionisable polymers. In this context, hydrophilic means that the polymer exhibits at least some moderate solubility or swellability in an aqueous medium having a pH in the range of gastrointestinal pH values, i.e. between pH 1 and pH 8. Neutral means that an aqueous solution of the polymer has an approximately neutral effect, at least in the absence of other solutes. Preferably, the neutral polymer is also a non-ionic polymer. Anionisable means that the polymer comprises moieties that readily deprotonate when dissolved in aqueous media except at an acidic pH. Some of the pharmaceutically acceptable hydrophilic polymers that are anionisable are sometimes also used as gastroresistant or enteric polymers. Moreover, the polymer is optionally a non-crosslinked polymer.

According to a further preferred embodiment, the pharmaceutically acceptable polymer in which the BK B2 receptor antagonist is amorphously and homogeneously dispersed is a thermoplastic polymer, i.e. a polymer that becomes pliable or mouldable at an elevated temperature and becomes solid again upon cooling down to e.g. room temperature. A thermoplastic polymer may also be understood as a polymer that goes through a glass transition temperature when heat is applied while maintaining chemical stability. In one embodiment, the polymer is a water-soluble (optionally pH-dependant), neutral (e.g. non-ionic or even non-ionisable) or anionisable, and thermoplastic.

Specific pharmaceutically acceptable polymers that may be selected include hydroxypropyl cellulose (HPC), hydroxypropyl methylcellulose (HPMC or hypromellose), hydroxypropyl methylcellulose acetate succinate (HPMCAS or hypromellose acetate succinate), povidone (PVP), copovidone (PVP/PA), polyethylene glycol (PEG), and polyethylene oxide (PEO). Each of these polymers may individually be used for forming the solid dispersion that incorporates the BK B2 receptor antagonist; alternatively, any combination of two or more of these polymers may be used, such as HPC in combination with HPMC, HPMC in combination with HPMCAS, PVP in combination with PVP/PA, or any other combination.

In one of the preferred embodiments, the pharmaceutically acceptable polymer is HPMCAS. HPMCAS, or hydroxypropyl methylcellulose acetate succinate, is an example of hydrophilic anionisable polymers that have a pH-dependent solubility in that it is insoluble in water at an acidic pH but soluble at a neutral or alkaline pH. HPMCAS may be understood as hydroxypropyl methylcellulose that has been esterified with a mixture of acetic acid and succinic acid. Generally, HPMCAS comprises from about 12% to about 28% of methoxy groups, from about 4% to about 23% of hydroxypropyl groups, from about 2% to about 16% acetyl groups, and about 4% to about 28% of succinoyl groups, calculated on a dry basis. HPMCAS is an exemplary pharmaceutically acceptable polymer, and also a thermoplastic polymer, as well as being hydrophilic and anionisable. Various grades of HPMCAS are available, including HPMCAS-LF which is characterised by a mean particle size in the range of about 5 µm and a viscosity in the range of about 3 mPa*s.

The inventors have found that the BK B2 receptor antagonist according to Formula (1) is not only surprisingly compatible with HPMCAS in that it forms solid dispersions at various useful weight ratios between the drug substance and the polymer; it was also discovered that the respective solid dispersions are highly stable, both chemically and physically; i.e. showing little chemical degradation and only a very low tendency for the drug substance to form crystals over time. Also surprisingly, solid dispersions of the compound of Formula (1) with HPMCAS exhibit very useful drug release profiles in spite of the pH-dependant solubility of the polymer.

In some of the preferred embodiments, the solid dispersion provided herein essentially consists of the pharmaceutically acceptable polymer, in particular HPMCAS, and the BK B2 receptor antagonist according to Formula (1), in particular (S)-N-(1-deutero-1-(3-chloro-5-fluoro-2-((2-methyl-4-(1-methyl-1H-1,2,4-triazol-5-yl)quinolin-8-yloxy)methyl)phenyl) ethyl)-2-(difluoromethoxy)acetamide. Various pharmaceutical grades of HPMCAS as described above may be used. In some preferred embodiments of HPMCAS, the content of acetyl groups is from of about 8% to about 12% and that of succinoyl groups is from about 7% to about 15%, resepctively. These polymer grades are typically insoluble in aqueous media at a pH below about 5.5 to about 6.5, and soluble at or above such pH. In some further preferred embodiments, the HPMCAS contains about 9% of acetyl groups and about 11% of succinoyl groups, and is water-soluble at a pH of about 6.0 and higher (currently commercially available e.g. as Aqoat^{®} AS-MMP). Moreover, an HPMCAS grade with a mean particle size in the range of about 200-300 µm is advantageous, but smaller particles sizes can also be used.

Alternatively, the solid dispersion may comprise copovidone (PVP/PA) as pharmaceutically acceptable polymer. It has been found that also this polymer enables the formation of advantageous solid dispersions incorporating the compound of Formula (1). These solid dispersions are also chemically and physically stable. One of their additional advantages is that they enable a high drug load with respect to the BK B2 receptor antagonist.

Copovidone, also referred to as copolyvidone, PVP/VA copolymer, or polyvinylpyrrolidone-vinyl acetate copolymer, is obtained by free-radical polymerization of vinylpyrrolidone and vinyl acetate in a molar ratio of about 6 : 4. Typical molecular weights are in the range of about 45,000 to 70,000. Examples of currently available commercial copovidone products include Kollidon VA 64 and Luviskol VA. Grades having different average particle sizes are also available.

In addition to the pharmaceutically acceptable polymers described above, one or more further polymers may be present in the solid dispersions. Moreover, a solid dispersion as claimed herein may also comprise a surfactant. In one embodiment, the surfactant is a non-ionic surfactant, in particular a non-ionic surfactant selected from the group of poloxamers and polysorbates. It is noted that these surfactants may also be considered as polymers; in other words, they may also function as contributing the effect of the polymeric drug carrier that forms the solid dispersion with the drug substance. They also function as surfactants in that they enhance the wetting of the solid dispersion upon contact with an aqueous medium such as water.

Poloxamers, also known as polyethylene-propylene glycol copolymers or polyoxyethylene-polyoxypropylene copolymers, are polyol block copolymers, each comprising a block of polyoxypropylene sandwiched between two blocks of polyoxyethylene. Various grades with different molecular weight ranges and different molecular ratios between the polyoxypropylene and polyoxyethylene blocks are available, such as poloxamer 124, 188, 237, 338 and 407.

The inventors have found that poloxamer 188 is particularly useful. It is a solid material with an average molecular weight in the range of about 7,680 to 9,510 in which the polyoxypropylene oxide block in average comprises about 27 repeating units and the polyoxyethylene blocks in average comprise about 80 repeating units. Particularly advantageous is poloxamer 188 in combination with copovidone.

Polysorbates, also referred to as polyoxyethylene sorbitan fatty acid esters, are another group of non-ionic surfactants which may be used as excipients in the formulation of the solid dispersions disclosed herein. Examples of potentially suitable polysorbates include polysorbate 20, 40, 60, 65, and 80, in particular polysorbate 20, which is also known as polyoxyethylene 20 sorbitan monolaurate and has an average molecular weight of 1,128, and polysorbate 80, whose chemical name is polyoxyethylene 20 sorbitan monooleate and which has a molecular weight of 1,310. A well-known branded product series is Tween.

Typically, the amount of the non-ionic surfactant in the solid dispersion, if present, is substantially lower than the amount of the polymer, e.g. the hydrophilic neutral or anionisable polymer, which does not represent a surfactant. Also typically, the amount of the non-ionic surfactant in the solid dispersion is lower than the amount of the active ingredient. In one specific embodiment, the amount of the non-ionic surfactant in the solid dispersion is in the range from about 0.5 wt.% to about 10 wt.%, based on the total weight of the solid dispersion, or from about 0.5 wt.% to about 5 wt.%. In a further specific embodiment, the weight ratio of the non-ionic surfactant to the polymer or, if a combination of two or more polymers is present, to the polymer combination, is in the range from about 1 : 100 to about 20 : 100, or from about 1 : 100 to about 10 : 100, respectively.

In some embodiments, the solid dispersion provided herein essentially consists of the pharmaceutically acceptable polymer, such as HPMCAS or copolyvidone, the BK B2 receptor antagonist according to Formula (1), in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, and optionally a non-ionic surfactant, such as a poloxamer or polysorbate.

In one of the preferred embodiments, the solid dispersion exhibits a content of the BK B2 receptor antagonist of at least about 10 wt.%, based on the total weight of the solid dispersion. As mentioned above, it is also a preferred embodiment according to which the BK B2 receptor antagonist of Formula (1) is the sole drug substance in the solid dispersion. Hence, the range of at least about 10 wt.% would also represent a preferred drug loading of the solid dispersion.

According to a further preferred embodiment, the solid dispersion exhibits a content of the BK B2 receptor antagonist of Formula (1) of about 10 wt.%, relative to the total weight of the solid dispersion. Further preferred is a solid dispersion with a content of the BK B2 receptor antagonist of Formula (1), in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, in the range of about 10 wt.% to about 50 wt.%, or from about 15 wt.% to about 50 wt.%, or from about 15 wt.% to about 45 wt.%, based on the total weight of the solid dispersion. Again, in a related embodiment, the solid dispersion exhibits a drug content in any of the above ranges, such as in the range of from about 15 wt.% to about 50 wt.%, wherein the BK B2 receptor antagonist of Formula (1), in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, represents the sole drug substance present in the solid dispersion. It was unexpected that such high drug loadings can be achieved for the compound of Formula (1) when following the guidance provided in this disclosure, without significant chemical or physical instability.

In a further preferred embodiment, the solid dispersion comprises about 15 wt.% to about 45 wt.%, such as about 15 wt.% to 25 wt% of the BK B2 receptor antagonist of Formula (1) and about 55 wt.% to 85 wt.%, such as about 75 wt.% to 85 wt.% of the polymer HPMCAS, based on the total weight of the solid dispersion. A related embodiment provides a solid dispersion essentially consisting of about 15 wt.% to 25 wt.% of the BK B2 receptor antagonist of Formula (1) and about 75 wt.% to 85 wt.% HPMCAS. For example, the solid dispersion may essentially consist of about 20 wt.% of the BK B2 receptor antagonist of Formula (1) in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, and about 80 wt.% HPMCAS. In further embodiments, the solid dispersion essentially consists of about 25 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and about 75 wt% HPMCAS; or about 30 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and about 70 wt.% HPMCAS, or about 35 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and about 65 wt.% HPMCAS, respectively.

It has been found by the inventors that the above ranges and the corresponding weight ratios between the drug substance and the polymer HPMCAS yield solid dispersions that exhibit a sufficiently high drug load to allow an effective and convenient dosing, a high product stability and shelf life, as well as desirable release profiles.

According to an alternative preferred embodiment, the solid dispersion comprises about 30 wt.% to 50 wt.% of the BK B2 receptor antagonist of Formula (1), about 50 wt.% to 70 wt.% copovidone, and optionally up to about 5 wt.% poloxamer, such as poloxamer 188, based on the total weight of the solid dispersion. Again, in a related embodiment according to which no other constituents are present in the solid dispersion, a solid dispersion is provided which essentially consists of about 30 wt.% to 50 wt.% of the BK B2 receptor antagonist of Formula (1), about 50 wt.% to 70 wt.% copovidone, and optionally up to about 5 wt.% poloxamer, such as poloxamer 188. For example, the solid dispersion may essentially consist of about 40 wt.% of the BK B2 receptor antagonist of Formula (1), about 58.5 wt.% of copovidone, and about 1.5 wt.% of poloxamer 188. These embodiments are advantageous in that they allow for a particularly high drug load, while at the same time being chemically and physically stable and providing useful drug release properties.

The solid dispersion may be provided in a physical form which is suitable for oral administration, or which is suitable for being incorporated in a pharmaceutical dosage form that is adapted for oral administration, such as a capsule or tablet. If the solid dispersion is designed to be administered as such, it may be provided as solid single unit, such as a melt-extruded form that is convenient to handle and swallow. Such product design may be particularly useful if an immediate release profile is desired, for example in case of acute therapies for spontaneously occurring symptoms. Since the BK B2 receptor antagonist, which has very poor solubility in physiological media, is provided in a solubilised format, it can be released substantially faster than from conventional solid dosage forms that typically contain the active ingredient in crystalline form.

In addition, the inventors have surprisingly discovered that the solid dispersions disclosed herein are not only useful for immediate release formulations, but also for extended-release products that release the BK B2 receptor antagonist over a prolonged period of time, as will be discussed in more detail below. For such products, it is particularly advantageous to provide the solid dispersion in a powder or granular form such as to facilitate its incorporation into a sustained release dosage unit such as a capsule or tablet.

Accordingly, in one of the preferred embodiments, the solid dispersion as provided by the inventors is in the form of a powder or granules. Both powders and granules provide the solid dispersion as multiple particles, i.e. in a multiparticulate format. In this context, the expressions "powder" and "granules" are to be understood simply as referring to the approximate particle sizes, wherein a powder refers to a relatively fine multiparticulate material and granules refer to a coarser material, regardless of the manufacturing method by which these materials were obtained. In other words, the expression "granules" should not be interpreted narrowly such as to require that a specific granulation method was used to produce the respective particles.

In a further aspect, the invention provides a method for preparing the solid dispersions described above. In some embodiments relating to this aspect, the method for preparing the solid dispersion comprises a step of hot-melt extrusion of a powder or granule mixture comprising the BK B2 receptor antagonist and the pharmaceutically acceptable polymer. In some other embodiments, the method comprises a step of spray drying an organic solution of the BK B2 receptor antagonist and the pharmaceutically acceptable polymer, or spraying of an organic solution of the active ingredient and the pharmaceutically acceptable polymer on particles of a pharmaceutically inactive material under conditions that cause the evaporation of the organic solvent(s). In this context, again, the BK B2 receptor antagonist is a compound according to Formular (1), in particular in a preferred form as disclosed above; and the polymer is the at least one pharmaceutically acceptable polymer in which the BK B2 receptor antagonist is homogeneously dispersed.

Hot-melt extrusion is a technique that is generally known in the field of pharmaceutics. It is also generally known that the technique may in principle be used to produce melt extrudates. This includes the equipment that may potentially be used for hot-melt extrusion as well as some of the common process parameters such as temperature and the rotation speed of screws in case that screw extruders are used. Based on the technical guidance with respect to the composition of the solid dispersions provided herein, and taking into account the working examples described below, a skilled person will be able to prepare the melt-extruded solid dispersions comprising the active ingredient according to Formula (1) without difficulty.

Hot-melt extrusion converts a powder or granule mixture comprising the BK B2 receptor antagonist of Formula (1) and the at least one pharmaceutically acceptable polymer into an extrudate which represents a solid dispersion in that the active ingredient becomes homogeneously and amorphously dispersed in the polymer. Hot-melt extrusion involves heating, softening or melting, mixing or homogenising, and extruding the mixture. It may also be described as a process whereby a blended composition is heated and/or compressed to a molten (or softened) state and subsequently forced through an orifice where the extruded product (extrudate) is formed into its final shape in which it solidifies upon cooling. The blended composition may be conveyed through one or more heating zones, which is typically achieved by a screw mechanism. The screw or set of screws may be rotated by a variable speed motor inside a cylindrical barrel where only a small gap exists between the outside diameter of the screw and the inside diameter of the barrel. In this conformation, high shear is created at the barrel wall and between the screw flights by which the components of the powder or granule blend are thoroughly mixed and disaggregated while being transported through the extruder. An extrudate refers to a composition formed by hot melt extrusion.

In one embodiment, the hot-melt extrusion step comprises the following substeps: (i) adding the optionally crystalline BK B2 receptor antagonist of Formula (1) (or e.g. a hydrate thereof) and the pharmaceutically acceptable polymer to a hot melt extrusion device; (ii) melting the BK B2 receptor antagonist and the polymer at temperatures close to or above the melting point of the BK B2 receptor antagonist and substantially less than its decomposition temperature; (iii) mixing the BK B2 receptor antagonist and the polymer to form a homogenous blend; (iv) extruding the homogenous blend to form the solid dispersion shaped as an extrudate. Steps (ii) and (iii) may occur simultaneously, or step (iii) may begin as soon as the optionally crystalline BK B2 receptor antagonist of Formula (1) and the polymer begin to melt. Again, the BK B2 receptor antagonist starting material in step (i) may be crystalline, and subsequently convert to the amorphous form during the hot melt extrusion process.

In one of the preferred embodiments, the step of hot melt extrusion is performed with a powder or granule mixture comprising about 15 wt.% to 45 wt.%, such as about 15 wt.% to 25 wt.% of the BK B2 receptor antagonist and about 55 wt.% to 85 wt.%, such as about 75 wt.% to 85 wt.% HPMCAS, based on the total weight of the powder or granule mixture. In a related embodiment, a powder or granule mixture essentially consisting of about 15 wt.% to 45 wt.%, or about 15 wt.% to 25 wt.%, such as about 20 wt.%, of the BK B2 receptor antagonist, in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, and about 55 wt.% to 85 wt.%, or about 75 wt.% to 85 wt.%, such as about 80 wt.% of HPMCAS is subjected to hot-melt extrusion such as to obtain a melt extrudate that represents a solid dispersion. In further embodiments, the powder subjected to hot-melt extrusion essentially consists of about 25 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and about 75 wt.% HPMCAS; or about 30 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and about 70 wt.% HPMCAS, or about 35 wt.% of (S)-N-(1-deutero-1-(3-chloro-5-fluoro-2-((2-methyl-4-(1-methyl-1H-1,2,4-triazol- 5-yl)quinolin-8-yloxy)methyl)phenyl)ethyl)-2(difluoromethoxy)acetamide and about 65 wt.% HPMCAS, respectively.

In another one of the preferred embodiments, the step of hot-melt extrusion is performed with a powder or granule mixture comprising about 30 wt.% to 50 wt.% of the BK B2 receptor antagonist, about 50 wt.% to 70 wt.% copovidone, and optionally up to 5 wt.% of poloxamer, in particular poloxamer 188, based on the total weight of the powder or granule mixture. In a related embodiment, powder or granule mixture consists of about 30 wt.% to 50 wt.% (such as about 40 wt.%) of the BK B2 receptor antagonist, about 50 wt.% to 70 wt.% (such as about 58.5 wt.%) copovidone, and optionally up to 5 wt.% (such as about 1-2 wt.%) of poloxamer, in particular poloxamer 188. For the avoidance of doubt, in these and the previous embodiments, the weight percentages are based on the weight of the powder or granule mixture that is subjected to the hot melt extrusion step.

The extrusion temperature, in some preferred embodiments, is selected to be above the glass transition temperature (T_{g}) of the respective carrier polymer or, if more than one carrier polymer is used, above the T_{g} of the carrier polymer having the highest T_{g}. In related preferred embodiments, the extrusion temperature is also selected above the melting temperature of the active ingredient For example, the extrusion at a temperature of at least about 20 °C above the melting point of the API would facilitate solubilisation of the drug substance in the carrier polymer(s). In other preferred embodiments, the extrusion is carried out at a temperature of at least about 20 °C above the T_{g} of the polymer. In practise, extruders may have several temperature zones that may be set at different temperatures; in this context, the extrusion temperature should be understood as the temperature of the extruder zone with the highest temperature.

As mentioned, the solid dispersions as disclosed herein may also be prepared using a method that comprises a step of spray drying an organic solution of the BK B2 receptor antagonist and the polymer, i.e. the at least one pharmaceutically acceptable polymer in which the BK B2 receptor antagonist according to Formula (1) is amorphously and homogeneously dispersed. Prior to the actual spray drying step, an organic solution of the BK B2 receptor antagonist according to Formula (1) and the polymer (and any other constituents of the solid dispersion which may optionally be incorporated) in an organic solvent which is pharmaceutically suitable for the spray drying process and which is capable of fully dissolving both the active ingredient and the polymer must be prepared, which can be done by any convention method of adding granular or powdery solids to an organic solvent under conditions (e.g. stirring, or elevated temperature) which lead to the dissolution of the solids.

Organic solvents that may be used in this context include, for example, methanol, ethanol, isopropanol, acetone, dichloromethane, chloroform, dimethyl formamide, dimethyl sulfoxide, ethyl acetate as well as any mixtures thereof. Optionally, the solvent or solvent mixture may contain limited amounts of water.

The spray drying step itself may be described as atomization under conditions that allow the evaporation of the organic solvent. For example, the atomization can be conducted by a nozzle or in a rotating disk. A further drying step may be used subsequently, such as fluidized bed drying, vacuum drying, tray drying, microwave drying, drum drying or double cone vacuum drying in order to reduce residual solvents to a pharmaceutically acceptable level. Generally, spray drying refers to making a highly dispersed liquid suspension or solution contact with air with enough volume, and causing evaporation and drying of droplets. Optionally, the organic solution of the BK B2 receptor antagonist according to Formula (1) and the polymer is injected into warm filtered air or inert gas flow to evaporate the solvent: The dried product particles may be conveyed to an accumulator, such as a cyclone particle collector.

Typically, the step of spray drying is performed at a temperature (i.e. inlet temperature) in the range of about 40°C to 150°C, or from about 50°C to 150°C, depending on the organic solvent that is used. In some embodiment, the inlet temperature is from about 80°C to 120°C. The outlet temperature for spray drying is typically lower, such as from about 40°C to 100°C, or from 40°C to 75°C. such as fluidized bed drying (such as from about room temperature to about 100°C), vacuum drying, (such as from about room temperature to about 200°C), such as from about room temperature to about 65°C, such as about 45°C.

In addition to methods relying on hot melt extrusion or spray drying, several other techniques or methods may also be used for the preparation of the solid dispersions disclosed herein. These methods include, for example, cryogenic processing techniques, freeze drying, fluid-bed coating, microwave irradiation, co-precipitation, electrostatic spinning, supercritical anti-solvent precipitation, spray congealing, spray coating of carrier particles, melt agglomeration, and others.

As previously mentioned, the solid dispersions as disclosed herein may be provided in the form of a powder or granules. Both powders and granules provide the solid dispersion as multiple particles, i.e. in a multiparticulate format. Such powders or granules may be obtained directly from the technique that is applied for preparing the solid dispersions. For example, if spray drying or a method involving a spray drying step is used for preparing the solid dispersions, the immediate product obtained from such method is already in the form of a powder or granulate. Optionally, a subsequent step to adjust the particle size (distribution) of the product may be conducted, such as sieving, milling, or agglomeration. In case a method comprising a step of hot melt extrusion is used, the product directly obtained from the extrusion step is typically not a powder or granulate, but in the form of larger units or elongated cylindrical rods which may subsequently be cut or pelletised into smaller units. A tool for cutting or pelletising the extrudate may be incorporated as a downstream module of the extrusion equipment If further diminution is required in view of a desired particle size, an independent subsequent milling step may used, optionally followed by a classifying step, such as sieving.

In a further preferred embodiment, the solid dispersion is in the form of a powder or granules, and prepared by a method comprising a hot melt extrusion step as described above, and a milling step wherein the extrudate is converted into the powder or granulate, optionally with an intermediate step of cutting or pelletising the extrudate prior to milling. The milling may be conducted using any pharmaceutically suitable equipment, such as a hammer mill, centrifugal impact mill, jet mill, ball mill, cone mill, oscillating and rotating sieve mill to name only a few examples. Optionally, the milling occurs at controlled temperature and/or under cooling and feeding. The milling process may itself include screening or classifying the milled particles, depending on the milling method and the type of equipment used. Alternatively, a separate screening or classifying step may be performed after milling. The milling of the extrudate, optionally including a subsequent screening step, may be conducted such as to achieve an average particle size, for example, in the range of about 50 µm to 800 µm, or in a further preferred range of about 100 µm to 700 µm, respectively. In this context, the average particle size typically means the D50 value of the powder or granulate as measured by laser diffraction. Alternatively, if the particle size distribution is characterised by sieve analysis, the more relevant parameter is the mass median sieve diameter, which is also in the range of about 50 µm to 800 µm, or of about 100 µm to 700 µm, or of about 150 µm to 500 µm, according to some preferred embodiments.

A further aspect of the invention relates to the use of the solid dispersion described above, in particular if provided in the form of a powder or granules. In general, the solid dispersion may be used for preparing any type of pharmaceutical composition, e.g. by incorporating the solid dispersion along with optional pharmaceutical excipients into pharmaceutical dosage forms. In some preferred embodiment, the solid dispersion is used for oral dosage forms, in particular for solid single-unit oral dosage forms, such as tablets, capsules (such as two-piece hard capsules), oral films, oral granules (such as single-unit sachets or stick packs), and the like.

A pharmaceutical composition, in the context of the present invention, should be understood as a composition that is technically suitable for being administered as a medicament to a subject, such as a human patient. It is composed, formulated and processed in compliance with general pharmaceutical standards, as may be defined for example in the official pharmacopeias or guidances issued by the regulatory agencies such as the FDA, PMDA and the EMA. In one of the preferred embodiments, the composition is adapted for oral administration, which implies, for example, that the excipients used, including their grades and their amounts, are safe and acceptable for oral use, in particular for oral administration to a human subject.

One of the unique properties of the solid dispersions found by the inventors is that they can advantageously be used for oral immediate release compositions as well as for oral extended-release compositions. Incorporated within immediate release compositions, the solid dispersions enable quick dissolution of the BK B2 receptor antagonist of Formula (1), which is a poorly soluble compound that otherwise (i.e. when provided in crystalline form) dissolves very slowly and incompletely in the commonly used dissolution testing models, which translates into poor and erratic or variable systemic absorption from the gastrointestinal tract after oral administration of a dose unit of a conventional immediate release composition comprising the BK B2 receptor antagonist in crystalline form, e.g. in the form of the crystalline monohydrate. As such, the solid dispersions when used in immediate release compositions are useful for increasing the extent of oral bioavailability of this active ingredient, for reducing the variability of the bioavailability or of the plasma level profiles, and also for achieving a more rapid absorption and thereby an accelerated onset of action compared to a conventional formulation comprising the drug in crystalline form.

In this context, an immediate release composition or formulation is an oral pharmaceutical composition designed to release most of its active ingredient over a period 30 minutes in an accepted *in vitro* dissolution model, including the models disclosed in the United States Pharmacopeia (USP), general chapter <711> ("DISSOLUTION"). More specifically, an immediate release composition may be described as a composition fulfilling the criteria, in particular the dissolution acceptance criteria, as established by the major regulatory bodies, such as provided in the applicable FDA Guidances that are generally known to a person skilled in the art.

Immediate-release oral dosage forms using the solid dispersion may, for example, be designed as immediate-release tablets. Tablet formulations that rapidly disintegrate upon contact with water are generally known to those skilled in the art. Incorporating the BK B2 receptor antagonist as active ingredient in the form of the solid dispersion will not only ensure that the tablet disintegrates quickly, but also that it rapidly, completely and reliably released the active ingredient in dissolved form. Similarly, the solid dispersion may be incorporated in capsules, such as hard gelatin capsules or other two-piece capsules, optionally together with further excipients as may be required or useful.

Moreover, the solid dispersion may be used in granule formulations which may, for example, be used as a so-called sprinkle-on formulations because these granules are typically administered by sprinkling on semisolid food, such as apple sauce, for ingestion. In particular, this is a suitable dosage form design for paediatric uses. Optionally, the solid dispersion may be provided for this purpose as coated granules, wherein the coating is typically a non-functional coating and primarily designed to improve product characteristics that are not related to drug release, such as the appearance or the swallowability of the granules.

In general, immediate-release formulations comprising the solid dispersion are very useful for acute treatment, or as on-demand medication for acute or chronic conditions, for example, in situations where symptoms appear or in which the severity of symptoms increases. In "acute treatment" or "on-demand treatment", it is imperative to immediately halt the progression after the onset of symptoms, in order to alleviate, abate or reduce the severity and/or duration of at least one of the symptoms and/or prevent additional symptoms.

A further preferred embodiment relates to the use of the solid dispersion described above in the preparation of a pharmaceutical composition for the extended-release of the BK B2 receptor antagonist. The inventors have found that the incorporation of the solid dispersion in such extended-release composition is unexpectedly advantageous, in spite of the fact that the solid dispersion itself has drug solubilising properties which would not be expected to be useful for providing slow drug release over an extended period of time. However, it was found that the solid dispersion is remarkably compatible with formulation techniques for extended-release, such as matrix tablets, and allows the creation of extended-release profiles over long periods of, e.g., about 8 to 24 hours. Moreover, the solid dispersions when used in extended-release formulations help to achieve reliable release profiles. Moreover, it was found that the use of the solid dispersion extended-release formulations is associated with a low or moderate impact of food on the overall exposure (e.g. indicated by the AUC), contrary to what would normally have been expected for extended-release compositions of the BK B2 receptor antagonist of Formula (1), in view of its solubility and intrinsic release behaviour.

As used in this context, an extended-release composition is any pharmaceutical composition or formulation adapted for oral administration which exhibits extended-release of the active ingredient Extended-release may also be referred to as slow release, prolonged-release, controlled-release, or sustained-release, to mention only a few alternative expressions, without limitation.

In a further aspect, accordingly, the present invention relates to a pharmaceutical extended-release composition comprising the solid dispersion disclosed hereinabove. Again, the preferences and optional features described for the solid dispersion are also applicable for this aspect. For example, in one of the preferred embodiments, the solid dispersion comprised in the pharmaceutical extended-release composition comprises the BK B2 receptor antagonist of Formula (1) wherein R is deuterium. As another example for a preferred embodiment of the pharmaceutical extended-release composition, the composition contains a solid dispersion according to any one of the preferred embodiments disclosed above, such as a solid dispersion comprising about 15 wt.% to about 45 wt.%, such as about 15 wt.% to about 25 wt.% of the BK B2 receptor antagonist of Formula (1), in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, and about 55 wt.% to about 85 wt.%, such as about 75 wt.% to about 85 wt.% HPMCAS, or a solid dispersion comprising about 30 wt.% to about 50 wt.% of the BK B2 receptor antagonist, about 50 wt.% to about 70 wt.% copovidone, and optionally up to about 5 wt.% poloxamer, respectively.

With respect to the type of dosage form used for the extended-release composition, the inventors have found that tablets and capsules are particularly advantageous. In one of the preferred embodiments, the extended-release composition is in the form of a matrix tablet

As used in the context of oral extended-release products, a matrix tablet is a pharmaceutical tablet wherein the active ingredient (incorporated as such or in the form of formulated particles or granules) is incorporated within an excipient matrix which is adapted for providing slow release. Such matrix tablet is different, for example, from a tablet whose extended-release functionality is predominantly effected by a sustained release coating. An extended-release matrix tablet may also comprise a coating, but typically this coating has little effect on the drug release profile. For example, a so-called non-functional coating may be applied to a matrix tablet, such coating being primarily used for improving the appearance of the tablet, facilitating its handling and swallowing, covering the taste, or reducing friability.

Formulation techniques for extended-release matrix tablets are generally known to those skilled in the art. Depending on the primary mechanism of drug release, erodible matrix tablets may be distinguished from "insoluble" matrix tablets that release their active ingredient mainly by diffusion out of a swollen but intact matrix. In practice, it is believed that often a combination of release mechanisms are simultaneously present, with some drug release occurring by diffusion while other portions of the drug are released as a consequence of tablet erosion. As used herein, erosion comprises any gradual loss of matrix material, regardless of the mechanism by which that loss occurs.

In one preferred embodiment, the matrix tablet comprises about 10 wt.% to about 60 wt.% of the solid dispersion, based on the total weight of the matrix tablet In further preferred embodiments, the content of the solid dispersion in the matrix tablet is in the range of about 15 wt.% to about 50 wt.%, in particular in the range from about 20 wt.% to about 40 wt%, such as about 20 wt.%, about 25 wt.%, about 30 wt.%, about 35 wt.%, or about 40 wt.%, respectively.

The matrix tablet may incorporate the solid dispersion in the form of a powder or granules, such as in the form of a milled extrudate as described above. The inventors have surprisingly found that relatively high amounts of the solid dispersions described herein can be incorporated into tablets prepared by direct compression, i.e. in the absence of an intermediate granulation step that is often required for incorporating poorly compressible materials into tablets. This was particularly unexpected for solid dispersions prepared by hot-melt extrusion followed by pelletisation and milling, which often leads to particles that have poor compressibility. Accordingly, in some further preferred embodiments, the matrix tablet comprises from about 15 wt.% to about 50 wt.%, or from about 20 wt.% to about 40 wt.%, of a solid dispersion in the form of a milled extrudate.

In addition to the solid dispersion, the matrix tablet preferably comprises further excipients, such as may be necessary or useful for tablet compression, but and also for the purpose of forming a matrix in which the solid dispersion powder or granules are embedded. The incorporation of powders or granules into compressed tablets may change certain characteristics of the solid dispersion as described above. For example, once a powder or a granulate has been incorporated within a compressed tablet, the flowability that typically characterises a powder or granules may not be present any longer. Moreover, even if particles representing the solid dispersion may, with some technical difficulty, be extracted from the matrix tablet, the particle size-related parameters may no longer be the same as before the solid dispersion was incorporated in the tablet. In this respect, the embodiments relating to matrix tablets comprising the solid dispersion with the optional or preferred features described in the context of the solid dispersion should be understood as also covering matrix tablets that are obtainable from powder or granule mixtures incorporating such solid dispersions by compressing these mixtures.

As mentioned, the matrix tablet comprises the solid dispersion, and preferably also comprises one or more further excipients, e.g. for the purpose of imparting compressibility and/or enabling the formation of a matrix having extended-release properties.

In some preferred embodiments, the matrix tablet comprises a tablet filler. A tablet filler may be understood as a pharmaceutical excipient enhancing the general processability of powder or granule mixtures in view of tablet compression. The functions of a tablet filler may include those of diluents or bulking agents, compression aids, and dry binding agents (often simply referred to as binders). Tablet fillers are also referred to as tablet filler-binders. Such excipient may be a compound or a combination of compounds. Potentially suitable tablet fillers to be used in the matrix tablet include, without limitation, microcrystalline cellulose, starch such as corn starch, potato starch or pregelatinised starch; mannitol, lactose, sorbitol, cellulose, calcium sulfate, calcium phosphate such as dibasic calcium phosphate anhydrous, erythritol and calcium carbonate. In one of the preferred embodiments, the tablet filler comprises microcrystalline cellulose. In a related embodiment, the tablet filler consists of microcrystalline cellulose, or of a combination of microcrystalline cellulose with dicalcium phosphate, such as a combination of microcrystalline cellulose with dicalcium phosphate at a weight ratio of about 1 : 1.

In a further preferred embodiment, the tablet further comprises at least one excipient that is useful for forming a matrix capable of embedding the solid dispersion powder or granules and effecting sustained release of the active agent Such excipient may be referred to as a matrix-forming excipient. In a further embodiment, this excipient is a matrix-forming polymer, in particular a hydrophilic matrix-forming polymer. Such hydrophilic matrix-forming polymers are, when compressed into tablets and upon contact with water or aqueous media, capable of swelling or gelling, and subsequently eroding slowly over time, i.e. over at least several hours. Example of potentially suitable hydrophilic matrix-forming polymers that may be used for formulating the matrix tablet include, without limitation, hydroxypropyl cellulose (HPC), hydroxypropyl methylcellulose (HPMC), alginic acid, bentonite, carbomer, carboxymethylcellulose calcium, carboxymethyl cellulose sodium, carrageenan, cellulose, copovidone, gelatin, guar gum, hydroxyethylcellulose, hydroxypropyl starch, hydroxypropyl methylcellulose (hypromellose), hydroxypropyl methylcellulose acetate succinate, methylcellulose, pectin, polyethylene oxide, polymethacrylates, potassium (or calcium, or sodium) alginate, povidone, tragacanth, or xanthan gum. In one of the preferred embodiments, the hydrophilic matrix-forming polymer in the matrix tablet is, or comprises, hydroxypropyl methylcellulose, or a combination of two or more grades of HPMC. In a related embodiment, the matrix tablet comprises a combination of two or more hydrophilic matrix-forming polymers, preferably including hydroxypropyl methylcelluloses.

According to further preferred embodiments, the matrix tablet comprises a glidant and/or a lubricant Also preferred is a related embodiment according to which the matrix tablet comprises a hydrophilic matrix-forming polymer, a tablet filler, a glidant and a lubricant

A glidant is an excipient that improves the flow properties powders or granulates by decreasing interparticle friction and cohesion. Potentially suitable glidants include, without limitation, silicon dioxide, in particular colloidal silicon dioxide, anhydrous colloidal silica, peptized silica, talc, magnesium trisilicate, or powdered cellulose. In one embodiment, the glidant is colloidal silicon dioxide.

In the context of pharmaceutical tablet compression, a lubricant may be understood as an excipient which decreases the friction between the surface of a powder, granulate or tablet on the one hand and the wall of the die of a tablet press and the tablet punch on the other hand. Thus, a lubricant reduces wear and tear of tablet dies and punches. Potentially suitable lubricants include, without limitation, magnesium stearate, polyoxyethylene glycol, aluminum stearate, calcium stearate, calcium behenate, castor seed oil, talc, sodium benzoate, glyceryl mono fatty acid, glyceryl monostearate, glyceryl dibehenate, glyceryl palmito-stearic ester, hydrogenated cotton seed oil, sodium lauryl sulfate, sodium stearyl fumarate, fumaric acid, and stearic acid.

In an alternative embodiment, the solid dispersion, if provided in the form of a powder or granulate, may be used for making extended-release pellets or -granules. For example, the particles of the solid dispersion (powder or granules) may be coated with an excipient or excipient mixture capable of providing for extended-release of the BK B2 receptor antagonist of Formula (1). The excipients may be applied to the solid dispersion by dry powder layering, granulation or agglomeration, or wet coating, such as aqueous or organic film coating. A related embodiment is directed to extended-release pellets or -granules comprising the solid dispersion embedded in, or coated with, an excipient or excipient mixture providing extended-release. Potentially suitable excipients and coating or agglomeration techniques are generally known to those skilled in the art.

Moreover, such extended-release pellets or granules incorporating the solid dispersion comprising the BK B2 receptor antagonist according to Formula (1) may be advantageously used for making tablets, such as fast disintegrating tablets which upon disintegration release the extended-release pellets or granules; or for filling hard capsules, also known as two-piece capsules, such as hard gelatin capsules on demand treatment or similar capsules made of non-gelatin materials. Accordingly, in some embodiments, the pharmaceutical extended-release composition comprising the solid dispersion is a tablet or a hard capsule comprising extended-release pellets or granules.

In one of the preferred embodiments, the BK B2 receptor antagonist of Formula (1) is the only active ingredient in the pharmaceutical composition. This should also be understood as a general preference for pharmaceutical compositions in the context of the present invention. Alternatively, the pharmaceutical composition may comprise one or more further active ingredients. The further or supplemental active ingredient or active pharmaceutical ingredient is preferably an active agent or active pharmaceutical ingredient which has utility in the prevention or treatment of one or more condition(s) responsive to BK B2 receptor modulation, including a condition selected from the group comprising a skin disorder; eye disease; ear disease; mouth, throat and respiratory disease; gastrointestinal disease; liver, gallbladder and pancreatic disease; urinary tract and kidney disease; disease of male genitale organs and female genitale organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema. For instance, at least one compound or pharmaceutically acceptable salt of the invention may advantageously be contained in a combination preparation that includes an antibiotic, anti-fungal, or anti-viral agent, an anti histamine, a non-steroidal anti-inflammatory drug, a disease modifying anti-rheumatic drug, a cytostatic drug, a drug with smooth muscle activity modulatory activity, an antibody, or mixtures of the aforementioned as further or supplemental active agent or active pharmaceutical ingredient.

The extended-release compositions may further be described in terms of the dose strength or amount of the BK B2 receptor antagonist of Formula (1) that is incorporated per dose unit, i.e. per capsule or tablet. In some embodiments, the dose strength is is the range from about 1 mg to about 100 mg, or from about 5 mg to about 100 mg, respectively. In further preferred embodiments, the dose strength is in the range from about 10 mg to about 80 mg, such as about 10 mg, 20 mg, 30 mg, 40 mg, 50 mg, 60 mg, 70 mg, or 80 mg, respectively. In some further preferred embodiments, the dose strength is in the range from about 20 mg to 50 mg, such as from about 20 mg to 40 mg. For paediatric use, also lower doses such as from about 0.5 mg to about 5 mg may be useful.

As already mentioned, the inventors have found that the incorporation of the solid dispersions comprising the BK B2 receptor antagonist of Formula (1) in extended-release compositions as disclosed herein allows the tailoring of clinically advantageous extended-release profiles with, for example, good robustness and reliability, and with a reduced food effect, especially with respect to the absorbed amount of drug, compared to conventional formulation techniques. This advantage is particularly pronounced for extended-release compositions comprising solid dispersions using hydroxypropyl methylcellulose acetate succinate (HPMCAS) as the carrier of the solid dispersion, i.e. as the pharmaceutically acceptable polymer in which the active ingredient is amorphous and homogeneously dispersed. Without wishing to be bound by theory, these release properties may relate to the fact that HPMCAS is a polymer that is insoluble in acidic media (e.g. gastric fluid) such as to cause a slow drug release, if any, in such acidic environment in which the drug substance shows at least some appreciable solubility; whereas the compound of Formula (1) itself is very poorly soluble in particular in relatively neutral media (e.g. intestinal fluid) such as to cause a relatively slow drug release in such environment, so that the various pH-dependent effects on the drug release rate may be partially compensated. In other words, the design of an extended-release composition comprising the solid dispersion based on HPMCAS may effect a controlled drug release even in response to pH changes. In some preferred embodiments, the extended-release composition as disclosed herein is an optionally coated single-layer tablet. A single-layer tablet consists of a single compressed tablet layer, which is different from bilayer or multilayer tablets. The manufacture of single-layer tablets is generally more cost-effective than that of tablets having two or more layers. Single-layer tablets may also be smaller and thus easier to swallow than tablets having one or more additional layers which are sometimes used to tailor the drug release profile.

Extended-release profiles, while also aiming at describing the behaviour of a composition or formulation in the relevant biological environment, i.e. in the clinical situation after administration of the composition or formulation to a subject, are typically measured in well-defined *in vitro* models. As used herein, an extended-release profile means the dissolution profile of an extended-release composition. A description of some widely accepted dissolution models for determining dissolution profiles is found, for example, in the United States Pharmacopeia (USP), general chapter <711> ("DISSOLUTION"). For characterising the dissolution profiles of the extended-release compositions disclosed herein, the Apparatus II (paddle apparatus) described in the USP may, for example, be used. The rotation speed of the paddle may, according to one preferred embodiment, be set at 100 rpm. The medium to be used for dissolution testing may, for example, consist of 1,000 mL of USP phosphate buffer pH 6.8 with 0.3 % (w/v) sodium dodecyl sulphate.

In some preferred embodiments, the extended-release compositions comprising the solid dispersions as disclosed herein exhibit a release of the BK B2 receptor antagonist of Formula (1) over a period of at least about 6 hours, or over at least about 8 hours, such as over about 8 to 24 hours. In a further preferred embodiment, the extended-release compositions release the BK B2 receptor antagonist over a period of 12 to 24 hours, as determined by dissolution testing of the composition using apparatus II of USP, chapter <711>, at 37 °C and at a rotation speed of 100 rpm, using 1,000 mL of USP phosphate buffer pH 6.8 with 0.3 % (w/v) sodium dodecyl sulphate as dissolution medium, and using tablet sinkers (e.g. Japanese sinkers) to keep the tablets in the bottom region of the dissolution vessels. In this context, the period of 12 to 24 hours over which the active ingredient is released means that the point in time when 95% of the active ingredient incorporated in the composition has been released falls within the specified time period. For example, the extended-release composition may be characterised by a dissolution profile which reaches 95% drug dissolution after about 12 hours, or after about 13, 14, 15, 16, 17, 18, 19 , 20, 21, 22, 23 or 24 hours, respectively.

The extended-release profiles, or dissolution profiles, may also be described in terms of the duration of time until about 50% of the incorporated drug released, i.e. dissolved. In some of the preferred embodiments, the extended-release compositions reach 50% dissolution within a period of about 3 to 10 hours, or within a period of about 4 to 8 hours, such as after about 4, 5, 6, 7 or 8 hours, respectively.

In some of the preferred embodiments, the extended-release compositions have a dose strength in the range from about 10 mg to 80 mg and release the BK B2 receptor antagonist of Formula (1) over a period of about 12 to 24 hours, as previously defined. Also preferred are embodiments according to which the extended-release compositions are in the form of matrix tablets comprising a solid dispersion, based on, or incorporating, HPMCAS, wherein the tablets have a dose strength of about 20 mg to 40 mg and exhibit a release over a period of about 12 to 24 hours.

Any references to methods of treatment by therapy or surgery or in vivo diagnosis methods of this description is to be interpreted as a reference to compounds, pharmaceutical compositions and medicaments of the present invention for use in those methods. According to a further aspect of the invention, the solid dispersion as disclosed herein, or the extended-release composition comprising such solid dispersion, may be used in the treatment of a subject suffering from any disease or condition responsive to bradykinin B2 receptor modulation. Such condition, including a condition selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema. Expressed in other words, one aspect of the invention relates to a method of treating a subject suffering from any disease or condition responsive to bradykinin B2 receptor modulation, wherein the method comprises the administration of the solid dispersion or the extended-release composition as described above. Similarly, the invention provides the use of the solid dispersion or the extended-release composition in the manufacture of a medicament for the treatment of any disease or condition responsive to bradykinin B2 receptor modulation.

As used herein, the expressions "treatment", "treating" and the like should be interpreted to include any type of prophylactic or therapeutic treatment As such, it encompasses the prevention, management or therapy of a disease or its reoccurrence, or of any symptoms associated with such disease or condition. Treatment includes acute and chronic treatment

Chronic treatment, in the context of the invention, refers to continuous treatment of a disease or condition, comprising repeated administration of a medication according to a regular dosing regimen over an extended period of time, such as over at least one month, or over at least three months. In certain embodiments wherein the condition of the patient or subject does not improve, the compound of Formula (1) is administered chronically, that is, for an extended period of time, including throughout the duration of the life of the patient or subject in order to ameliorate or otherwise control or limit symptoms associated with a disease or condition in the patient or subject In further preferred embodiments, the treatment may be prophylactic treatment, *i.e.,* before the onset of symptoms, in order to prevent, delay or reduce the severity of symptoms. Prophylactic treatment can include both, acute and chronic treatment For example, acute prophylactic treatment may include orally administering to the human patient or subject in need thereof at least once a therapeutically effective dose of the compound of Formula (1) to thereby prevent or alleviate symptoms. Chronic treatment may, for example, include the administration of one, two or three dose units per day over such period.

As previously mentioned, according to one of the preferred embodiments, an extended-release composition is provided that comprises the solid dispersion of the BK B2 receptor antagonist of Formula (1) in a polymeric carrier; of which the inventors have found that it provides advantageous extended-release properties. It is believed that these properties also render such extended-release compositions particularly suitable for chronic, or long-term treatment One of the specific benefits provided by these compositions is that they provide sustained effective and tolerable plasma levels with a regimen of only one or two dosings per day. Accordingly, in some of the preferred embodiments, the solid dispersion or the extended-release composition disclosed herein is used in the chronic treatment of a subject suffering from a disease or condition responsive to BK B2 receptor modulation, preferably over a period of at least one month. In further embodiments, the chronic treatment is characterised by one or two dosings per day, preferably over a period of at least three months, or of at least one year, respectively. In one embodiment, the chronic treatment is lifelong treatment

For the avoidance of doubt, the respective treatment durations do not require that the same dosing regimen is used throughout the entire treatment. For example, if once-daily dosing becomes insufficient to treat a specific subject, the regimen may be switched to twice daily administration. Alternatively, the dose per administration event may be increased within chronic treatment.

Moreover, in the context of the invention, "acute" includes any non-chronic administration regimen, for example a single administration of a composition comprising the solid dispersion disclosed herein, or an immediate release composition comprising the solid dispersion, as well as a sporadic or regular dosing regimen over a relatively short period of time, such as up to four weeks, or up to two weeks. In some embodiments, the solid dispersion provided by the invention is used for the acute treatment of a disease or condition responsive to bradykinin B2 receptor modulation.

The following diseases or conditions may be considered as responsive, or at least potentially responsive, to bradykinin B2 receptor modulation:
Skin disorders include, without limitation, disorders such as skin aging, skin efflorescences including pressure sores, decubital ulcers, irritated, sensitive and dysaesthetic skin, erythema, rash, skin edema, psoriasis, eczema, lichen, bacterial, viral, fungal and parasites induced skin infections including furuncle, abscess, phlegmon, erysipelas, folliculitis and impetigo, lice, scabies and herpes simplex, acne, exanthema, dermatitis including atopic dermatitis, allergic contact dermatitis, neurodermatitis, radiation damage, sunburn, pruritus, itching, urticaria, psoriasis, mycosis, tissue ulceration, epidermolysis bullosa, wounds including abnormal wound healing, burns, frostbite, skin inflammation and edema caused by venoms, alopecia, hair squama, corn, wart and panaris.

Eye diseases include, without limitation, inflammatory disorders such as scleritis, conjunctivitis, chemosis, iritis, iridocyclitis, uveitis, chorioretinitis, as well as disorders such as retinochoroidal circulatory disorders, bacterial eye infections, unspecific conjunctivitis and eye irritations, retinopathy of prematurity, proliferative vitreoretinopathy, macular degeneration (including age related macular degeneration and including both wet and dry forms), corneal diseases including corneal graft rejection, corneal injury, corneal scarring, corneal ulceration, corneal haze, keratoconus, glaucoma (preferably open angle glaucoma), myopia, ocular hypertension, ocular vessel damage, angiogenesis, eye fibrosis (e.g. anterior subcapsular fibrosis, posterior subcapsular opacities, posterior capsular opacities, corneal haze after laser surgery, subconjunctival scarring after glaucoma surgery), proliferative vitreoretinopathy (PVR), bacterial ocular infections including hordeolum and ptilosis.

Ear diseases encompass, but are not limited to, disorders such as Meniere's disease, inflammation of the middle ear, inflammation of the external auditory canal and acute hearing loss.

Mouth, throat and respiratory diseases comprise, without limitation, disorders such as inflammation of the oral mucosa and gums including aphta and stomatitis, parodontitis, epiglottitis, pharyngitis, laryngotracheitis, tonsillitis, common cold, angina, rhinitis including seasonal allergic rhinitis or perennial allergic rhinitis, rhinorrea, sinusitis of whatever type, etiology or pathogenesis or sinusitis that is a member selected from the group consisting of purulent or nonpurulent sinusitis, acute and chronic sinusitis and ethmoid, frontal, maxillary or sphenoid sinusitis, expectoration, pneumoconiosis of whatever type or genesis, including for example aluminosis, anthracosis, asbestosis, chalicosis, siderosis, silicosis, tabacosis and, in particular, byssinosis, bronchitis, cough, trachitis, congestion, pneumonia, eosinophilc lung infiltrate, chronic eosinophilic pneumonia, idiopathic pulmonary fibrosis and other fibrotic lung diseases, treatment related fibrotic lung disease e.g. related to radiation, methotrexate, chemotherapy, amiodarone or nitrofurantoin, sarcoidosis, acute respiratory distress syndrome (ARDS), bronchoconstriction, asthma of whatever type, etiology, or pathogenesis, or asthma that is a member selected from the group of atopic asthma, non-atopic asthma, allergic and non-allergic asthma, extrinsic asthma caused by environmental factors, intrinsic asthma caused by pathophysiologic disturbances, bronchial asthma, IgE-mediated asthma, essential asthma and essential asthma of unknown or inapparent cause, true asthma, emphysematous asthma, exercise-induced asthma, occupational asthma, infective asthma caused by bacterial, fungal, protozoal or viral infection, incipient asthma, wheezy infant syndrome, bronchial hyperreactivity, chronic obstructive pulmonary disease (COPD), COPD that is characterized by irreversible, progressive airways obstruction, acute respiratory distress syndrome (ARDS) and exacerbation of airways hyperreactivity consequent to other drug therapy, dyspnea, hyperoxic alveolar injury, pulmonary emphysema, pleurisy, tuberculosis, exposure to high altitude i.e. acute mountain sickness and preferably high altitude pulmonary edema (HAPE), resistant cough, bronchial hyporeactivity.

Gastrointestinal diseases include, without limitation, disorders including esophagitis, gastritis, irritable stomach, gastric and duodenal ulcer, ileus, colon irritable, inflammatory bowel diseases including Crohn's disease and ulcerative colitis, enteritis, hypertensive gastro- and colopathy, colitis, peritonitis, appendicitis, rectitis, gastrointestinal hemorrhage caused by a portal hypertension, collateral circulation or hyperemia, postgastrectomy dumping-syndrome, digestion discomfort, diarrhea, hemorrhoids, worm diseases, abdominal colic and colic of parts of the gastrointestinal system.

Liver, gallbladder and pancreatic diseases encompass, but are not limited to, disorders such as hepatitis, cirrhosis of the liver, liver fibrosis (e.g. due to viral (HBV/HCV) infections, toxins (alcohol), fatty liver, bile stasis, hypoxia), portal hypertension, hepatorenal syndrome, hepatogenic edema, cholangitis, cholecystitis, acute and chronic pancreatitis, and biliary colic.

Urinary tract and kidney diseases include, without limitation, urinary tract infections such as acute and chronic cystitis, interstitial cystitis, irritable bladder, overactive bladder, incontinence including but not limited to stress-, urge and reflex incontinence, benign prostate hyperplasia, chronic renal disease, urethritis, inflammatory kidney diseases including glomerulonephritis, glomerular disease of the kidney, interstitial nephritis, pyelonephritis, diuresis, proteinuria, natriuresis, calciuresis, disorders of water balance, disorders of electrolyte balance, disorders of acid-base balance and renal colic, renal fibrosis, chronic renal allograft dysfunction, contrast-induced nephropathy.

Diseases of male genitale organs and female genitale organs include, without limitation, altered sperm mobility, male infertility, orchitis, prostatitis, prostate enhancement, mastitis, inflammatory pelvis diseases, vaginal infections and pain, adnexitis, colpitis, soft ulcus, syphilis, clap and ovarian hyperstimulation syndrome.

Diseases of the hormone system include, without limitation, menstrual disorders and pain, climacteric disturbance, emesis, premature uterine contractions, premature labor, endometriosis, endometritis, myoma, pre-eclampsia.

Metabolic diseases include, without limitation, disorders such as diabetes, including non-insulin dependent diabetes mellitus, diabetic retinopathy, diabetic macular edema, diabetic nephropathy and diabetic neuropathy, insulin resistance and diabetic ulceration, diseases of the proteo- and purine metabolism such as gout and disorder of lipometabolism, hypoglycemia.

Cardiovascular diseases include, without limitation, disorders including vascular permeability, vasodilation, peripheral circulatory disorders, arterial circulatory disorders including aortic aneurysm, abdominal aortic aneurysm, brain aortic aneurysm, hypertension and hypotension associated with sepsis, restenosis after percutaneous transluminal coronary angioplasty, atherosclerosis including atherosclerotic plaque rupture, hemangioma, angiofibroma, venous disorders such as thrombosis, varicosity, phlebitis, thrombophlebitis, phlebothrombosis, cardiopathy, congestive heart failure, coronary heart disease, carcinoid syndrome, angina pectoris, cardiac dysrhythmias, inflammatory heart diseases including endocarditis, pericarditis and constrictive pericarditis, myocarditis, myocardial infarct, postmyocardial infarction syndrome, left ventricular dilation, post ischemic reperfusion injury, shock and collapse including septic, allergic, post traumatic and hemodynamic shock, amniotic fluid embolism, systemic inflammatory response syndrome (SIRS) including SIRS caused by heartlung bypass during surgery, sepsis and internal and external complications during cardiopulmonal bypass surgery (including but not limited to adverse hemodynamic effects following protamine sulfate reversal of heparin.

Blood diseases include, without limitation, disorders such as coagulation, disseminated intravascular coagulopathy, hemorrhage, hemorrhagic diathesis, hypercholesterolemia and hyperlipemia, hypovolemic shock, paroxysmal nocturnal haemoglobinuria.

Lymphatic diseases include, without limitation, splenomegaly, lymphangitis, lymphadenitis and hyperplastic adenoids.

Disorders of the central nervous system include, without limitation, disorders such as inflammatory diseases of the central nervous system including encephalitis, meningitis, encephalomyelitis, meningoencephalitis, hydrocephalus, amyotrophic lateral sclerosis, spinal cord trauma, spinal cord edema, demyelinating diseases of the nervous system, multiple sclerosis, acute and chronic neuro-degenerative disorders including aging, Alzheimer's disease and Parkinson's disease, neuritis, and peripheral neuropathy, depressions, anorexia, anxiety and schizophrenia, sleep disorders.

Brain disorders include, without limitation, disorders such as nootropic or cognition enhancement, cerebral amyloid angiopathy, stroke, head and brain trauma, traumatic brain injury, brain tumor, cerebral heat damage, cerebral ischemia, cerebral hemorrhage, post traumatic and post ischemic cerebral edema, general brain edema, acute mountain sickness and preferably high altitude cerebral edema (HACE), cytotoxic brain edema, vasogenic brain edema, post-surgical brain edema, brain edema associated with metabolic diseases, increase of permeability of blood-brain barrier or blood-brain tumor barrier.

Musculoskeletal system diseases include, without limitation, disorders such as inflammatory musculoskeletal disorders, arthrosis, osteoarthrosis, osteoarthritis, chondroporosis after joint trauma or relatively long immobilization of a joint after meniscus or patella injuries or torn ligaments, rheumatoid arthritis of whatever type, etiology, or pathogenesis including acute arthritis, acute gouty arthritis, chronic inflammatory arthritis, degenerative arthritis, infectious arthritis, Lyme arthritis, proliferative arthritis, vertebral arthritis, septic arthritis, psoriatic arthritis, chronic polyarthritis, rheumatism, Sjogren's syndrome, lumbago, spondylitis, spondylarthritis, ankylosing spondylitis, osteomyelitis, sprain, teno-synovitis, inflammation-induced bone resorption, fracture or the like, osteoporosis, musculoskeletal pain and hardening, spinal disk syndrome.

Allergy disorders include, without limitation, disorders such as general allergic reactions, food allergy, anaphylactic shock, allergic contact hypersensitivity, allergic skin reactions, allergic asthma, vernal conjunctivitis and seasonal or perennial allergic rhinitis.

Pain includes, without limitation, centrally and peripherally mediated pain, vascular pain, visceral pain, inflammatory mediated pain, neuralgic pain, referred pain, nociceptive pain, reflectory pain, psychosomatic pain, acute pain such as caused by acute injury, trauma or surgery of bones, muscle, tissue, soft tissue, organs, pain after insect bites, post-stroke pain syndrome, post-surgery pain, progressive disease related pain, chronic pain such as caused by neuropathic pain conditions (including but not limited to complex regional pain syndrome, causalgia, morbus sudeck, reflex sympathetic dystrophy, diabetic peripheral neuropathy, post-herpetic neuralgia, trigeminal neuralgia, cancer-related pain, pain associated with rheumatoid arthritis, osteoarthritis, teno-synovitis, gout, menstruation and angina, fibromyalgia, ocular pain, back pain, headache, cluster headache, migraine, inflammatory pain, which may be associated with acute inflammation or chronic inflammation. Inflammatory pain includes but is not limited to neuropathic pain, ischemic pain, pain induced by arthritis, muscle pain induced by acute or chronic inflammation, neuralgia caused by acute or chronic inflammation, hyperalgesia. Also chemotherapy-induced peripheral neuropathy, hyperalgesia, opioid-induced hyperalgesia and fever. Furthermore, compounds of the invention are useful as analgesic agent for use during general and monitored anesthesia.

Infectious diseases include, without limitation, diseases including those mediated by bacteria, viruses, fungi, parasites, protozoa, prions or mycobacterial infections. Particularly, the present invention is useful for the treatment of bacterial infections caused by Streptococcus, Escherichia, Salmonella, Staphylococcus, Klebsiella, Moracella, Haemophilus and Yersinia. Examples of bacterial infections intended to be within the scope of the present invention include, but are not limited to diseases such as pestis, sepsis, epidemic typhus, food poisoning, tetanus, scarlet red, whooping cough, diphtheria. Examples of viral infections intended to be within the scope of the present invention include, but are not limited to diseases such chickenpox and herpes zoster, AIDS, influenza, small pox, and children diseases such as measles, rubella, mumps, acute anterior poliomyelitis. The present invention is useful for the treatment of protozoa and parasites infections caused by Schistosoma mansoni, Dermatofagoides farinae, and Plasmodium inducing Malaria. Examples of prion infections intended to be within the scope of the present invention include but are not limited to diseases such bovine spongiform encephalopathy (BSE), Creutzfeldt Jacob disease and kuru, dengue fever, hemorrhagic fever.

Inflammatory disorders include, without limitation, disorders such as acute-phase reaction, local and systemic inflammation and inflammation caused by other diseases whatever type, etiology or pathogenesis and caused by those inflammatory diseases specified within this application.

Injuries: Within the present application the term "injuries" encompasses, but is not limited to, multiple trauma, head trauma, lung injuries, external, internal and surgery wounds.

Immunology disorders include, without limitation, disorders such as hyperesthesia, autoimmune disorders, graft rejection in transplantation, transplant toxicity, granulomatous inflammation / tissue remodelling, myasthenia gravis, immunosuppression, immune-complex diseases, over- and underproduction of antibodies, vasculitis, delayed graft function, lupus.

Cancers include, without limitation, disorders such as solid tumor cancer including breast cancer, lung cancer (non-small-cell lung cancer and small-cell lung cancer), prostate cancer, cancers of the oral cavity and pharynx (lip, tongue, mouth, pharynx), esophagus, stomach, small intestine, large intestine, colon, rectum, gallbladder and biliary passages, pancreas, larynx, lung, bone, osteosarcoma, connective tissue, skin cancer including Kaposi's syndrome, melanoma and skin metastasis, epidermoid cancer, basal cell carcinoma, cervix uteri, corpus endometrium, cancer of ovary, testis, bladder, ureter and urethra, kidney, eye, brain and central nervous system, pseudotumor cerebri, sarcoma, sarcoid, thyroid and other endocrine glands (including but not limited to carcinoid tumors), Hodgkin's disease, non-Hodgkin's lymphomas, multiple myeloma, hematopoetic malignancies including leukemias and lymphomas including lymphocytic, granulocytic and monocytic lymphomas, tumor invasion, metastasis, ascites, tumor growth and angiogenesis.

Hereditary diseases include, without limitation, disorders such as hereditary angioedema and angioneurotic edema, chondrocalcinosis, Huntington's disease, mucoviscidosis.

Edema, as used herein, includes, but is not limited to, general edema and edema caused by inflammation, Factor XII deficiency-induced edema, other drugs, e.g. drug induced angioedema, including but not limited to angiotensin-converting enzyme inhibitor-induced angioedema, infection, burns, injuries, trauma, frostbite, surgery, distorsions, fractures, exposure to high altitude (e.g. high altitude pulmonary edema (HAPE) and high altitude cerebral edema (HACE)), hereditary, autoimmune and other diseases and disorders, particularly but not limited to those disorders specified in this application, stress-induced edema (pronounced swelling) of gut. Known forms of angioedema (AE) include hereditary angioedema (HAE), acquired angioedema (AAE), bradykinin-mediated non-histaminergic idiopathic angioedema, allergic angioedema, and drug induced angioedema. The HAE can be of any type, including type I HAE, type II HAE, or type III HAE.

Capillary leak syndrome(s) include(s), without limitation, systemic capillary leak syndrome in sepsis, burn, allergy, drug/toxin-induced conditions, organ transplantation and IL-2 cytokine therapy.

In a further preferred embodiment, the solid dispersion or the extended-release composition is used in the treatment of AE, including HAE, AAE, bradykinin-mediated non-histaminergic idiopathic AE, allergic AE, and drug induced AE. AE is an area of swelling of the lower layer of skin and tissue just under the skin or mucous membranes. The debilitating and often painful swelling may occur in the face, lips, tongue, limbs, genitals, gastrointestinal mucosa, urogenital region and airways. Often it is associated with hives, which are swelling within the upper skin. It is characterized by repetitive episodes of swelling, and onset is typically over minutes to hours. Predicting where and when the next episode of angioedema will occur is impossible. Patients may have one episode per month, but there are also patients who have weekly episodes or only one or two episodes per year.

In some preferred embodiments, the solid dispersion or the extended-release composition is used in the treatment of HAE of any type, including type I HAE, type II HAE, or type III HAE, preferably type I HAE or type II HAE. HAE is a disorder that manifests in recurring attacks of severe swelling. The swelling often affects the arms, legs, face, intestinal tract, and also the airways. If the intestinal tract is affected, abdominal pain and vomiting may occur. Swelling of the airways may result in its bronchial obstruction and breathing difficulties. Acute attacks typically last for several days, and HAE patients experience attacks at a frequency of about every two weeks.

The extended-release composition is particularly advantageous in the chronic therapy of AE, such as hereditary angioedema (HAE) and acquired angioedema (AAE), and in particular type I HAE or type II HAE. As mentioned, it is patient-friendly in that it only requires the administration of a single daily dose or, alternatively, only two daily doses. In view of the robustness of the extended-release profiles with respect to pH-variations and the presence of food, these compositions are suitable for long-term therapy over periods of months or even years.

Further aspects of the invention, embodiments, optional features and preferences will become clear on the basis of the Examples and the patent claims.

Further definitions
For clarity, some further definitions of terms are given which are used throughout the description and claims. The definitions should be used to determine the meaning of the respective expressions unless the context requires a different meaning.

The terms 'a' or 'an' do not exclude a plurality, i.e., the singular forms 'a', 'an' and 'the' should be understood as to include plural referents unless the context clearly indicates or requires otherwise. In other words, all references to singular characteristics or limitations of the present disclosure shall include the corresponding plural characteristic or limitation, and vice versa, unless explicitly specified otherwise or clearly implied to the contrary by the context in which the reference is made. The terms 'a', 'an' and 'the' hence have the same meaning as 'at least one' or as 'one or more' unless defined otherwise. For example, reference to 'an ingredient' includes mixtures of ingredients, and the like.

The terms 'about' or 'ca.' will compensate for variability allowed for in the pharmaceutical industry and inherent in pharmaceutical products, such as differences in content due to manufacturing variation and/or time-induced product degradation. The term allows for any variation, which in the practice of pharmaceuticals would allow the product being evaluated to be considered bioequivalent in a subject to the recited strength of a claimed product

The terms 'active agent', 'therapeutic agent', 'active pharmaceutical ingredient (API)', 'active principle', 'drug', 'bioactive agent', are used synonymously and refer to a compound or combination of compounds which are pharmaceutically active against an undesired condition.

The term 'composition' refers to any type of composition in which the specified ingredients may be incorporated, optionally along with any further constituents.

The term 'compound' refers to a chemical substance, which is a material consisting of molecules having essentially the same chemical structure and properties. For a small molecular compound, the molecules are typically identical with respect to their atomic composition and structural configuration. For a macromolecular or polymeric compound, the molecules of a compound are highly similar but not all of them are necessarily identical.

The terms 'comprise', 'comprises' and 'comprising' and similar expressions are to be construed in an open and inclusive sense, as 'including, but not limited to'.

The terms 'essentially', 'about', 'approximately', 'substantially' and the like in connection with an attribute or value include the exact attribute or the precise value, as well as any attribute or value typically considered to fall within a normal range or variability accepted in the technical field concerned. For example, 'essentially free of water' means that no water is deliberately included in a composition but does not exclude the presence of residual moisture.

The term 'essentially consisting of' refers to compositions or dosage forms where no further components are added other than those listed. Nevertheless, very small amounts of other materials may potentially be present, such as material inherent impurities, or minor additives added by excipient providers. Furthermore, when referring to e.g., 'essentially consisting of A, B, C and optionally D.' this means that no further components are added to a composition or dosage form other than A, B, C and D, with D being an optional component (i.e., not mandatory) in said composition or dosage form.

The term 'essentially free' refers to a composition that contains less than a functional amount of the respective ingredient, typically less than 1 % by weight, preferably less than 0.1 % or even 0.01 %, and including zero percent by weight of the respective ingredient.

Some aspects of the invention are further illustrated by the following Examples, which should however not be understood as restricting the scope of the invention.

### EXAMPLES

The compound of Formula (1) was prepared as described in WO 2019/101906 and, where necessary, under Good Manufacturing Practice (GMP) regulations.

The studies reported in Examples 6 and 7 were carried out in accordance with corresponding protocols, existing guidelines and recommendations on laboratory animal science, current guidelines on Good Clinical Practice (GCP) of the International Council for Harmonisation of Technical Requirements for Pharmaceuticals for Human Use (ICH), and applicable regulatory and country-specific governmental rules for conducting animal and clinical trials, including rules on the protection of subjects and informed consent, and transparency requirements. All trial protocols, suitability of the investigator(s), facilities and the methods and material to be used in obtaining and documenting informed consent of the trial subject were reviewed and approved by the Institutional Review Board (IRB) or Independent Ethics Committee (IEC) before a study was started. For this study, a qualified person performed the final release of the study drug, i.e. the compound of formula (I), according to Directive 2003/94/EC annex 13; and study drug labels contained information to meet the applicable regulatory requirements. Trial medication was packed, labelled and released under the responsibility of the pharmacist of the clinical site in accordance with GMP practice guidelines, International Conference on Harmonization (ICH), Good Clinical Practice (GCP) and applicable local laws/regulations.

### Example 1: Solid dispersions prepared by vacuum melt compression

Two solid dispersion compositions were prepared by vacuum melt compression. The first one, designated as HME1, consisted of 40 wt.% of (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide, 58.5 wt.% copovidone (Kollidon^{®} VA64), and 1.5 wt.% of poloxamer 188. The second composition, designated as HME2, consisted of 20 wt.% (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide and 80 wt.% of hypromellose acetate succinate (AQOAT^{®} AS-MMP).

The active ingredient, (*S*)-*N*-(1-deutero-1-(3-chloro-5-fluoro-2-((2-methyl-4-(1-methyl-1*H-*1,2,4-triazol-5-yl)quinolin-8-yloxy)methyl)phenyl)ethyl)-2-(difluoromethoxy)acetamide, was provided as crystalline monohydrate in powder form. The measured amounts of the active ingredient and the carrier were mixed for 4 minutes in a shaker mixer (Turbula^{®}).

Subsequently, measured amounts of the powder mixtures were transferred into a lab-scale vacuum compression moulding apparatus (VCM Meltprep) equipped with a disc moulding tool (Meltprep Disc Tool) and molded into discs at a vacuum of -1.0 bar. For HME1, the vacuum compression moulding occurred at a temperature of 200 °C, using a heating time of 5 min, a cooling time of 2 min and a cooling air pressure of 2.5 bar. For HME2, the vacuum compression moulding occurred at a temperature of 180 °C, using a heating time of 5 min, a cooling time of 5 min and a cooling air pressure of 3.0 bar. With both compositions, transparent yellowish discs were obtained.

The discs were crushed with a mortar and a pestle for further assessment. Firstly, it was confirmed by an HPLC assay that the drug content was within the range of 90 to 110 % of the theoretical amount Moreover, the crystalline state of the drug substance was assessed by differential scanning calorimetry (DSC) and X-ray powder diffraction (XRPD, also referred to as powder X-ray diffraction or PXRD). The DSC thermograms showed no melting point of the active ingredient (around 111°C), only the Tg of the polymers were observable. The XRPD analysis indicated no residual crystallinity. Therefore, it was concluded that the samples represented solid dispersions comprising the active ingredient solely in the amorphous, fully dissolved state.

### Example 2: Solid dispersions prepared by spray drying

Four solid dispersion compositions, designated as SDD1 through SDD4, were prepared by spray drying, each consisting of 25 wt.% of (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide and 75 wt.% of carrier polymer. The carrier polymers were hypromellose acetate succinate (AQOAT^{®} AS-MMP) for SDD1, hypromellose of type 2910 USP (Methocel^{®} E3) for SDD2, copovidone (Kollidon^{®} VA64) for SDD3, and povidone K-30 for SDD4, respectively.

For each composition, the measured amounts of the active ingredient and the respective carrier polymer were dissolved in a mixture of dichloromethane (DCM) and methanol (ratio 4 : 1 (w/w)). Spray drying was then performed using a lab-scale spray dryer Büchi B290), using the parameters listed in Table 1 below.

**Table 1**

| Parameter | Value |
|---|---|
| Nozzle | 2-Fluid nozzle, 1.5 mm cap, 0.7 mm tip |
| Glassware | High efficiency cyclone |
| Atomizing air flow rate | 40 L/min |
| Solution feed | 7-12 g/min |
| Inlet temperature | 57-58 °C |
| Outlet temperature | 35-36 °C |
| Blower (aspirator) | 100% |
| Wash solution | DCM/methanol (4: 1 v/v) |

The spray drying process yielded powders that were collected and subjected to a secondary drying step, using vacuum drying at 40 °C and a vacuum of -26.5 mm Hg over 20 hours.

The samples thus obtained were assessed using modulated differential scanning calorimetry (mDSC), XRPD, and thermogravimetric analysis (also thermal gravimetric analysis, TGA). Both XRPD and mDSC data confirmed that the four prototypes were amorphous and free of any detectable levels of crystalline material. The TGA thermogram showed an inflection in the slope of the weight loss step for the SDD4 prototype, indicating a small amount of residual solvent left in the sample. Overall, all prototypes were confirmed to represent homogenous solid dispersions comprising the active ingredient in amorphous form, and fully dissolved in the carrier polymer.

Samples of the spray dried solid dispersions SSD1 through SSD4 were stored at elevated temperature (40 °C) and retested by XRPD after three weeks. In result, all samples were amorphous and free of crystalline content.

### Example 3: Solid dispersion prepared by hot melt extrusion

The same composition as HME2, consisting essentially of 20 wt.% (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and 80 wt.% of hypromellose acetate succinate (AQOAT^{®} AS-MMP) (see Example 1), was used for preparing a solid dispersion by hot melt extrusion. The powder blend comprising the crystalline active ingredient and the carrier polymer was screened through a sieve having an aperture width of 500 µm. The blend was then extruded using a twin screw extruder (Leistritz ZSE 12 HP-PH) fitted with a 1.5 mm die. The maximum temperature of the kneading zones was within a range of 145 to 165 °C, except that the die zone was set at 200 °C. Clear extrudates of a slightly brownish colour were obtained and pelletised with a pelletiser. Subsequently, the pellets were milled using a hammer mill in two phases, the first one with a screen size of 1,000 µm, the second one with a screen size of 400 µm, at a mill speed of 6,500 rpm. The particle size distribution was then characterised by laser diffraction (Mastersizer^{®}). For two milled batches, the average particle size D50 was 262.3 µm and 249.1 µm, respectively. In a sieve analysis, it was found that 66.2 wt.% and 61.7 wt.% of the milled particles, respectively, were in the sieve fractions of 180 µm to 355 µm.

A further batch of the same composition (HME2) was prepared using the same hot melt extrusion, pelletisation and milling process as described above, except that in the final milling step, a hammer mill with a screen size of 200 µm was used. In result, an average particle size D50 of about 100 µm, i.e. in the range from 63 µm to 125 µm as determined by sieve analysis was obtained.

Samples of the HME2 solid dispersion were stored at accelerated temperature (40 °C) to test their chemical and physical stability. The drug content and the impurities were quantified by HPLC. XRPD was used to detect changes in the crystalline state, if any. The test results are provided in Table 2 below. They indicate that the active ingredient is chemically highly stable in the solid dispersion and remains in the amorphous form.

**Table 2**

| Time | Start | 3 months | 6 months | 6 months |
|---|---|---|---|---|
| Temperature | - | 40 °C | 25 °C | 40 °C |
| Assay* (wt%) | 98.5 | 99.5 | 98.4 | 98.3 |
| Total impurities (wt.%) | 0.85 | 0.89 | 0.94 | 0.93 |
| Crystallinity | None | None | None | None |

| | | | | |
|---|---|---|---|---|
| *100% means the theoretical amount of the API in the sample | | | | |

### Example 4: Immediate-release formulations comprising solid dispersion

The solid dispersion formulation HME1 was prepared by vacuum melt compression according to Example 1, and the solid dispersion HME2 was prepared by hot melt extrusion and subsequent milling as described in Example 3. Moreover, the solid dispersions SDD1 and SDD3 were prepared as described in Example 2. Each of the four solid dispersions were then mixed with additional excipients and compressed into tablets of 150 mg weight, using a lab-scale tablet press (Kilian Styl'One Evolution, one punch tablet press simulator). The composition of these tablets (4.1 through 4.4) is given in Table 3. It is noted that the additional excipients do not include a hydrophilic matrix-forming polymer.

**Table 3**

| Tablet formulation | 4.1 | 4.2 | 4.3 | 4.4 |
|---|---|---|---|---|
| Solid dispersion type | HME1 | HME2 | SDD1 | SDD3 |
| Components (per unit) | [wt%] | [wt%] | [wt.%] | [wt.%] |
| Solid dispersion | 5.0 | 10.0 | 8.0 | 8.0 |
| Lactose monohydrate (Tablettose^{®} 70) | 66.0 | 62.0 | 64.0 | 64.0 |
| Microcrystalline cellulose (Avicel^{®} PH-102) | 26.0 | 25.0 | 25.0 | 25.0 |
| Crospovidone (Kollidon^{®} CL) | 2.0 | 2.0 | 2.0 | 2.0 |
| Silicon dioxide colloidal (Aerosil^{®} 200) | 0.5 | 0.5 | 0.5 | 0.5 |
| Magnesium stearate (Ligamed^{®} MF-2-V) | 0.5 | 0.5 | 0.5 | 0.5 |
| Total | 100.00 | 100.00 | 100.00 | 100.00 |

### Example 5: Matrix tablets comprising solid dispersions

The solid dispersion composition HME2 was prepared as described in Example 3 and incorporated in two matrix tablet formulations (5.1 and 5.2) whose compositions are provided in Table 4.

**Table 4**

| Matrix tablet formulation | 5.1 | | 5.2 | |
|---|---|---|---|---|
| | 20 mg API | | 40 mg API | |
| Components (per unit) | [mg] | [wt%] | [mg] | [wt.%] |
| HME2 | 100.00 | 27.78 | 200.00 | 31.25 |
| Hypromellose type 2208 (Methocel^{™} K100LV DC2) | 95.00 | 26.39 | - | - |
| Hypromellose type 2910 (Methocel^{™} E4M) | - | - | 150.00 | 23.44 |
| Hypromellose type 2208 (Methocel^{™} K100M DC2) | - | - | 40.00 | 6.25 |
| Microcrystalline cellulose (Avicel^{®} PH-102) | 80.00 | 22.22 | 120.00 | 18.75 |
| Calcium hydrogen phosphate dihydrate (Emcompress^{®}) | 80.00 | 22.22 | 120.00 | 18.75 |
| Silicon dioxide colloidal (Aerosil^{®} 200) | 2.00 | 0.56 | 4.00 | 0.63 |
| Magnesium stearate (Ligamed^{®} MF-2) | 3.00 | 0.83 | 6.00 | 0.94 |
| Total | 360.00 | 100.00 | 640.00 | 100.00 |

As explained in the detailed description above, the hypromelloses in these formulations represent hydrophilic matrix-forming polymers. Microcrystalline cellulose and calcium hydrogen phosphate dihydrate function as tablet fillers, silicon dioxide colloidal primarily functions as glidant, and magnesium stearate primarily functions as lubricant

Weighed amounts of the raw materials were mixed in several subsequent steps in a tumble blender (Turbula^{®} 2F): First, the milled extrudate, the fillers and the hydrophilic matrix-forming polymer(s) were mixed, then the glidant was added and mixed, and finally the lubricant was added and mixed. The resulting powder mixture was compressed on a lab-scale, single-punch pharmaceutical tablet press (Kilian Styl'One Evolution) into white to off-white, oval, biconvex tablets (17 mm x 7.2 mm) with brownish spots, using a main compression force in the range of 15 kN to 40 kN. It was observed that the milled extrudates were readily compressible within the respective powder mixtures, even in the absence of a granulation step. The tablets had a mean thickness of about 5.7 mm, a mean resistance to crushing of about 110 N to 170 N and a friability of about 0.2 wt.% to 0.7 wt.%. An assay (HPLC-UV) indicated that the drug content was within the acceptable limits of 90% to 110% of the labelled amount Total impurities were below 1.0%.

The drug release profiles of tablets of prototypes 5.1 and 5.2 were characterised by dissolution testing according to Ph.Eur. 2.9.3 / USP <711> using apparatus II (paddle apparatus). The dissolution medium was 1000 mL of USP phosphate buffer pH 6.8 with 0.3 % (w/v) sodium dodecyl sulphate. The paddle rotation speed was set at 100 rpm. Japanese sinkers were used for preventing the flotation of the tablets. Samples were withdrawn at intervals and analysed by HPLC with respect to their API concentration, from which the dissolved amounts of drug were calculated. The dissolution profiles are provided in Table 5.

**Table 5**

| Matrix tablet formulation | 5.1 | 5.2 |
|---|---|---|
| | 20 mg API | 40 mg API |
| Sampling time [min] | API dissolved | API dissolved |
| 0 | 0.0 % | 0.0 % |
| 60 | 14.5 % | 18.0 % |
| 120 | 25.4 % | 24.2 % |
| 180 | 35.4 % | 30.3 % |
| 240 | 44.4 % | 36.7 % |
| 360 | 60.8 % | 50.4 % |
| 480 | 75.9 % | 63.9 % |
| 600 | 90.5 % | 76.1 % |
| 720 | 99.1 % | 89.1 % |
| 840 | 103.4 % | 93.9 % |
| 960 | 104.3 % | 98.4 % |
| 1080 | 103.9 % | 100.5 % |
| 1200 | 104.0 % | 102.3 % |
| 1320 | 104.0 % | 102.3 % |
| 1440 | 104.4 % | 103.1 % |

As the testing results illustrate, the matrix tablets incorporating a solid dispersion of the BK B2 receptor antagonist according to Formula (1) are capable of effecting extended-release of the active ingredient over prolonged periods of time, such as over about 12 hours or even longer. Matrix tablet 5.1 exhibits a steeper dissolution profile than tablet 5.2 *in vitro.*

### Example 6: Human PK study

The basic pharmacokinetic profiles of the matrix tablet versions 5.1 and 5.2 after single administration were assessed and compared to immediate release capsules in a clinical phase I study, both under fasted and fed (high-carbohydrate high-fat (HCHF) meal) conditions. The study was designed as a randomised, five period crossover study with 10 healthy volunteers. Table 6 lists key PK parameters found in the study.

**Table 6**

| | 5.1 | 5.2 | SMEDDS (fasted) | 5.1 | 5.2 |
|---|---|---|---|---|---|
| Parameter | fasted | fasted | | fed | fed |
| Cmax (mean) [ng/mL] | 89.8 | 111 | 272 | 128 | 160 |
| - SD of Cₘₐₓ [ng/mL] | 31.8 | 46.1 | 130 | 50.1 | 76.8 |
| - CV of Cₘₐₓ [%] | 35.4 | 41.5 | 47.8 | 39.1 | 48.1 |
| C₂₄ₕ (mean) [ng/mL] | 9.1 | 40.3 | 3.7 | 9.4 | 35.4 |
| - SD of C₂₄ₕ [ng/mL] | 6.9 | 25.5 | 4.2 | 8.3 | 36.1 |
| - CV of C₂₄ₕ (%) | 76.0 | 63.3 | 112.2 | 88.3 | 101.9 |
| tₘₐₓ (median) [h] | 4.0 | 9.0 | 1.0 | 6.0 | 11.0 |
| AUC₀₋₂₄ₕ (mean) [ng*h/mL] | 753 | 1427 | 896 | 941 | 1586 |
| - SD of AUC₀₋₂₄ₕ [ng*h/mL] | 314 | 644 | 510 | 565 | 795 |
| - CV of AUC₀₋₂₄ₕ [%] | 41.7 | 45.1 | 56.9 | 55.2 | 50.2 |
| AUC_{inf} (mean) [ng*h/mL] | 867 | 1896 | 937 | 1028 | 1955 |
| - SD of AUC_{inf} [ng*h/mL] | 408 | 899 | 556 | 572 | 1209 |
| - CV of AUC_{inf} [%] | 47.0 | 47.4 | 59.3 | 55.6 | 61.9 |

The data demonstrate that the matrix tablet formulations exhibit extended-release characteristics *in vivo,* as indicated e.g. by the tmax values. Prototype 5.2, which releases the active ingredient over a longer period of time *in vitro* compared to prototype 5.1 (see Example 5), also leads to a later tmax *in vivo,* both under fasted and fed conditions. Quite remarkably, the matrix tablets exhibit an overall drug exposure as the immediate release formulation (cf. AUC_{inf} values), from which it can be concluded that the active ingredient, in spite of its unfavourable physical properties, is reliably released and absorbed even from the more distal regions of the intestines.

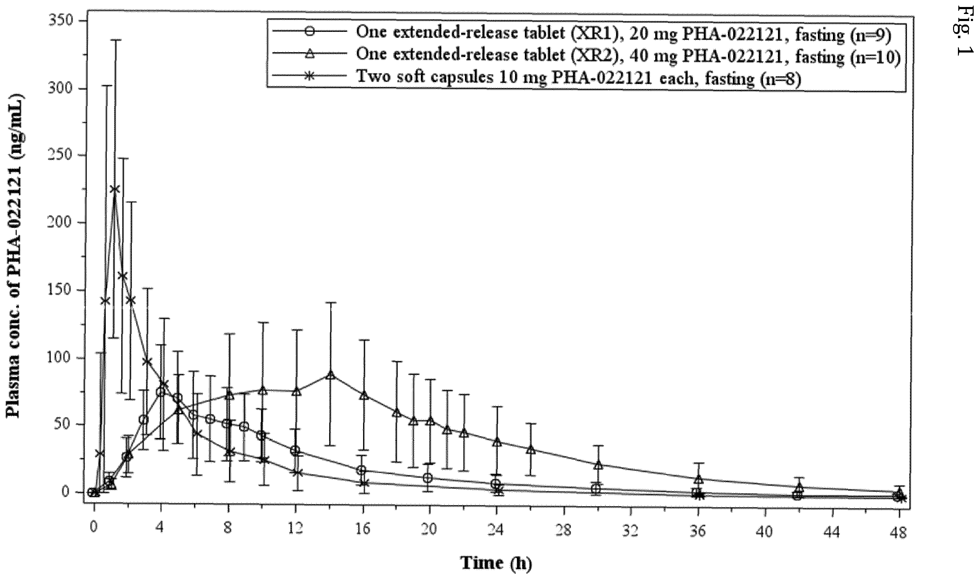

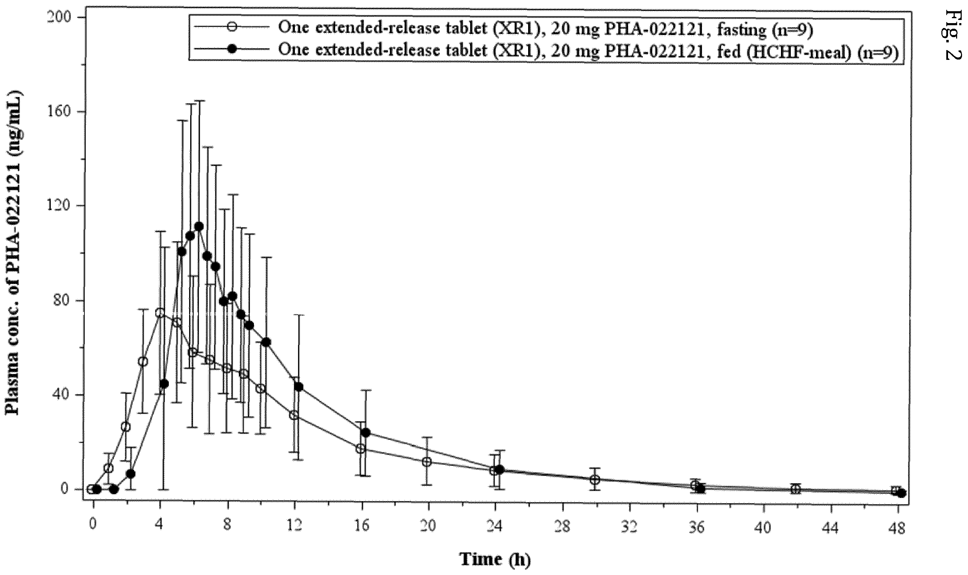

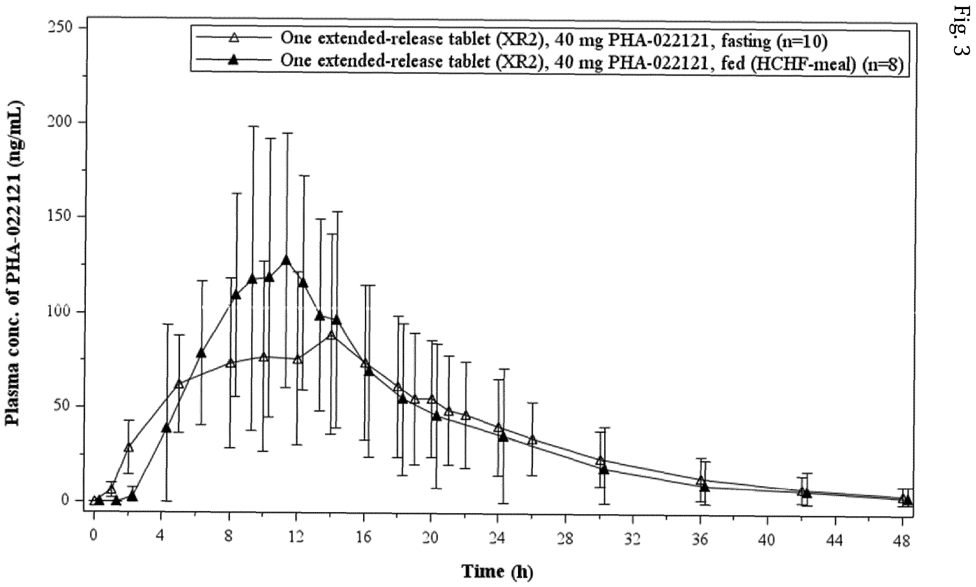

Figures 1 to 3 show the mean plasma concentration-over-time profiles achieved by the respective formulations: Figure 1 depicts the plasma profiles after administration in the fasting state, whereas each of Figures 2 and 3 shows a comparison of the mean plasma profiles after administration in the fed versus the fasting state for matrix tablet 5.1 (Figure 2) and matrix tablet 5.2 (Figure 3), respectively. Subsequent simulation studies confirmed the usefulness of the PK profiles of both tested matrix tablet formulations for continuous therapy using a once-daily or twice-daily dosing regimen. The SMEDDS formulation refers to immediate-release soft gelatin capsules comprising the active ingredient formulated as a self-microemulsifying drug delivery system (SMEDDS), by which a dose of 20 mg of the API was administered, as a comparator.

### Example 7: Monkey PK study

The solid dispersion-containing tablet formulations 4.1 through 4.4 were tested in male cynomolgus monkeys to assess their biopharmaceutical performance *in vivo.* For this purpose, they were placed in a size 0 hard gelatin capsules. Each capsule was pierced with a needle 10 times prior to administration. For comparison, a liquid self-microemulsifying drug delivery system (SMEDDS) of the same active ingredient (65 mg/mL) filled in hard gelatin capsules was administered. The actually administered doses of the active ingredient were in the range of 2.6 mg to 2.9 mg per dosing.

The study was designed as a three-phase cross-over study. In result, it was shown that the dose-normalised (DN) systemic exposure, expressed as DN AUC, did not differ between the formulations. In addition to such high extent of oral bioavailability, also the maximum plasma concentrations Cmax and the time to maximal plasma concentrations tmax indicated the suitability of the solid dispersion for immediate-release products and acute treatments. At the same time, it is noted that tablet 4.2 which incorporates the solid dispersion HME2 based on HPMCAS as the carrier polymer shows a delay in reaching maximum plasma concentrations, i.e. an increased tₘₐₓ value, which indicates a delayed release that is consistent with its pH-dependent aqueous solubility profile. Table 7 shows the key PK parameters as obtained in the study.

**Table 7**

| PK parameter | 4.1 | 4.2 | 4.3 | 4.4 | SMEDDS |
|---|---|---|---|---|---|
| DN Cmax (mean) [ng/mL] | 29.3 | 16.2 | 48.8 | 38.3 | 40.9 |
| - SD of DN Cmax [ng/mL] | 21.3 | 4.9 | 28.8 | 25.3 | 17.3 |
| tmax (median) [h] | 2.0 | 4.0 | 2.0 | 2.0 | 1.0 |
| DN AUCₗₐₛₜ (mean) [ng*h/mL] | 83.8 | 85.2 | 119.3 | 93.7 | 93.3 |
| - SD of DN AUCₗₐₛₜ [ng*h/mL] | 19.9 | 19.9 | 66.6 | 25.6 | 24.6 |

## Claims

1. A solid dispersion for oral administration comprising:
(a) a bradykinin (BK) B2 receptor antagonist having a chemical structure according to Formula (1), or a salt or solvate thereof: 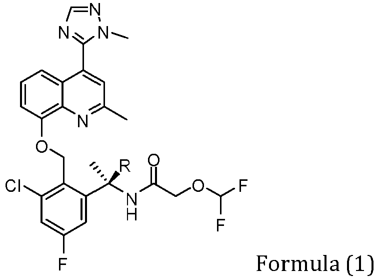 wherein R is deuterium or hydrogen, and
(b) at least one pharmaceutically acceptable polymer;
wherein the BK B2 receptor antagonist is amorphous and homogeneously dispersed in the polymer.

2. The solid dispersion of claim 1, wherein the BK B2 receptor antagonist is a compound according to Formula (1) with R being deuterium.

3. The solid dispersion of claim 1 or 2, wherein the pharmaceutically acceptable polymer is selected from hydroxypropyl cellulose (HPC), hydroxypropyl methylcellulose (HPMC), hydroxypropyl methylcellulose acetate succinate (HPMCAS), povidone (PVP), copovidone (PVP/PA), polyethylene glycol (PEG), and polyethylene oxide (PEO), and preferably from HPMCAS and copovidone (PVP/PA).

4. The solid dispersion of any one of the preceding claims, further comprising a non-ionic surfactant selected from the group of poloxamers and polysorbates.

5. The solid dispersion of any one of the preceding claims, wherein the content of the BK B2 receptor antagonist is in the range of 15 wt.% to 50 wt.%, based on the total weight of the solid dispersion.

6. The solid dispersion of claim 5, comprising
(i) about 15 wt.% to about 45 wt.% BK B2 receptor antagonist of Formula (1) and about 55 wt.% to about 85 wt.% HPMCAS, based on the total weight of the solid dispersion; or
(ii) 30 wt.% to 50 wt.% BK B2 receptor antagonist of Formula (1), 50 wt.% to 70 wt.% copovidone, and optionally up to 5 wt.% poloxamer, based on the total weight of the solid dispersion.

7. The solid dispersion of any one of the preceding claims, being in the form of a powder or granules.

8. A method for preparing the solid dispersion of any one of the preceding claims, comprising a step of (i) hot melt extrusion of a powder or granule mixture comprising the BK B2 receptor antagonist and the pharmaceutically acceptable polymer, or of (ii) spray drying of an organic solution of the BK B2 receptor antagonist and the pharmaceutically acceptable polymer.

9. The method of claim 8, wherein the step of hot melt extrusion is performed with
(i) a powder or granule mixture comprising about 15 wt.% to about 45 wt.% BK B2 receptor antagonist and about 55 wt.% to about 85 wt.% HPMCAS, based on the total weight of the powder or granule mixture; or
(ii) a powder or granule mixture comprising 30 wt.% to 50 wt.% BK B2 receptor antagonist and 50 wt.% to 70 wt.% copovidone, and optionally up to 5 wt.% poloxamer, based on the total weight of the powder or granule mixture

10. Use of the solid dispersion of any one of claims 1 to 7 in the preparation of a pharmaceutical composition for the extended-release of the BK B2 receptor antagonist

11. A pharmaceutical extended-release composition comprising the solid dispersion of any one of claims 1 to 7, wherein said composition is in the form of a matrix tablet, and wherein the matrix tablet comprises 20 wt.% to 40 wt.% of the solid dispersion, based on the total weight of the tablet

12. The extended-release composition of claim 11, wherein the matrix tablet further comprises a hydrophilic matrix-forming polymer and a tablet filler, and, optionally, a glidant and/or a lubricant, and wherein the hydrophilic matrix-forming polymer is HPMC, or a combination of two or more grades of HPMC.

13. The solid dispersion of any one of claims 1 to 7, or the extended-release composition of claim 11 or 12, for use in the treatment of a subject suffering from a disease or condition responsive to BK B2 receptor modulation, wherein the disease or condition responsive to BK B2 receptor modulation is selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema.

14. The solid dispersion or the extended-release composition for use according to claim 13, wherein the disease or condition responsive to BK B2 receptor modulation is angioedema, such as hereditary angioedema.

15. The solid dispersion or the extended-release composition for use according to claim 13 or 14, wherein the treatment is chronic treatment.

## Patentansprüche

1. Eine feste Dispersion zur oralen Verabreichung, umfassend:
(a) einen Bradykinin (BK)-B2-Rezeptorantagonisten, der eine chemische Struktur gemäß Formel (1) aufweist, oder ein Salz oder Solvat desselben: 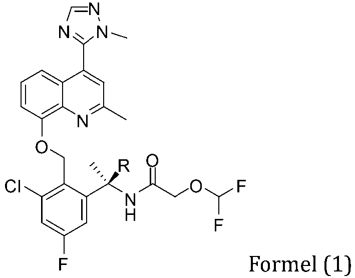 wobei R Deuterium oder Wasserstoff ist; und
(b) mindestens ein pharmazeutisch akzeptables Polymer;
wobei der BK-B2-Rezeptorantagonist amorph und homogen in dem Polymer dispergiert ist.

2. Die feste Dsipersion nach Anspruch 1, wobei der BK-B2-Rezeptorantagonist eine Verbindung gemäß Formel (1) ist, und wobei R Deuterium ist.

3. Die feste Dsipersion nach Anspruch 1 oder 2, wobei das pharmazeutisch akzeptable Polymer ausgewählt ist aus Hydroxypropylcellulose (HPC), Hydroxypropylmethylcellulose (HPMC), Hydroxypropylmethylcellulose-Acetat-Succinat (HPMCAS), Povidon (PVP), Copovidon (PVP/PA), Polyethylenglykol (PEG) und Polyethylenoxid (PEO), und vorzugsweise aus HPMCAS und Copovidon (PVP/PA).

4. Die feste Dsipersionnach einem der vorhergehenden Ansprüche, weiterhin umfassend ein nichtionisches Tensid, welches aus der Gruppe der Poloxamere und Polysorbate ausgewählt ist.

5. Die feste Dispersion nach einem der vorhergehenden Ansprüche, wobei der Gehalt des BK-B2-Rezeptorantagonisten in dem Bereich von 15 Gew.-% bis 50 Gew.-%, bezogen auf das Gesamtgewicht der festen Dispersion, ist.

6. Die feste Dispersion nach Anspruch 5, umfassend
(i) etwa 15 Gew.-% bis etwa 45 Gew.-% des BK-B2-Rezeptorantagonisten der Formel (1) und etwa 55 Gew.-% bis etwa 85 Gew.-% HPMCAS, bezogen auf das Gesamtgewicht der festen Dispersion; oder
(ii) 30 Gew.-% bis 50 Gew.-% des BK-B2-Rezeptorantagonisten der Formel (1), 50 Gew.-% bis 70 Gew.-% Copovidon, und optional bis zu 5 Gew.-% Poloxamer, bezogen auf das Gesamtgewicht der festen Dispersion.

7. Die feste Dispersion nach einem der vorhergehenden Ansprüche; wobei sie in Form eines Pulvers oder Granulats vorliegt.

8. Verfahren zur Herstellung der festen Dispersion nach einem der vorhergehenden Ansprüche, umfassend einen Schritt (i) der Heißschmelzextrusion eines Pulver- oder Granulatgemischs, das den BK-B2-Rezeptorantagonisten und das pharmazeutisch akzeptable Polymer umfasst, oder (ii) der Sprühtrocknung einer organischen Lösung des BK-B2-Rezeptorantagonisten und des pharmazeutisch akzeptablen Polymers.

9. Das Verfahren nach Anspruch 8, wobei der Schritt der Heißschmelzextrusion ausgeführt wird mit
(i) einem Pulver- oder Granulatgemisch, umfassend etwa 15 Gew.-% bis etwa 45 Gew.-% des BK-B2-Rezeptorantagonisten und etwa 55 Gew.-% bis etwa 85 Gew.-% HPMCAS, bezogen auf das Gesamtgewicht des Pulver- oder Granulatgemischs; oder
(ii) einem Pulver- oder Granulatgemisch, umfassend 30 Gew.-% bis 50 Gew.-% des BK-B2-Rezeptorantagonisten und 50 Gew.-% bis 70 Gew.-% Copovidon, und optional bis zu 5 Gew.-% Poloxamer, bezogen auf das Gesamtgewicht des Pulver- oder Granulatgemischs.

10. Verwendung der festen Dispersion nach einem der Ansprüche 1 bis 7 zur Herstellung einer pharmazeutischen Zusammensetzung zur verlängerten Freisetzung des BK-B2-Rezeptorantagonisten.

11. Pharmazeutische Zusammensetzung mit verlängerter Freisetzung, umfassend die feste Dispersion nach einem der Ansprüche 1 bis 7, wobei die Zusammensetzung in der Form einer Matrixtablette vorliegt, und wobei und die Matrixtablette 20 Gew.-% bis 40 Gew.-% der festen Dispersion, bezogen auf das Gesamtgewicht der Tablette, umfasst.

12. Die Zusammensetzung mit verlängerter Freisetzung nach Anspruch 11, wobei die Matrixtablette ferner umfasst: ein hydrophiles matrixbildendes Polymer und einen Tablettenfüllstoff, und, optional, ein Gleitmittel und/oder Schmiermittel, und wobei das hydrophile matrixbildende Polymer HPMC oder eine Kombination aus zwei oder mehr HPMC-Typen ist.

13. Die feste Dispersion nach einem der Ansprüche 1 bis 7, oder die Zusammensetzung mit verlängerter Freisetzung nach Anspruch 11 oder 12, zur Verwendung bei der Behandlung eines Subjekts, das unter einer Erkrankung oder einem Zustand leidet, die/der auf die Modulation des BK-B2-Rezeptors anspricht, wobei die Erkrankung oder der Zustand, die/der auf die Modulation des BK-B2-Rezeptors anspricht, aus der Gruppe, umfassend eine Hauterkrankung, eine Augenerkrankung, eine Ohrenerkrankung, eine Mund-, Rachen- und Atemwegserkrankung, eine Magen-Darm-Erkrankung, eine Leber-, Gallenblasen- und Bauchspeicheldrüsenerkrankung, eine Harnwegs- und Nierenerkrankung, eine Erkrankung der männlichen und weiblichen Geschlechtsorgane, eine Erkrankung des Hormonsystems, eine Stoffwechselerkrankung, eine Herz-Kreislauf-Erkrankung, eine Blutkrankheit, eine Erkrankung des Lymphsystems, eine Erkrankung des Zentralnervensystems, eine Erkrankung des Gehirns, eine Erkrankung des Muskel-Skelett-Systems, eine allergische Störung, Schmerzen, eine Infektionskrankheit, eine Entzündungskrankheit, eine Verletzung, eine immunologische Erkrankung, Krebs, eine Erbkrankheit und Ödem, ausgewählt ist.

14. Die feste Dsipersion oder die Zusammensetzung mit verlängerter Freisetzung zur Verwendung nach Anspruch 13, wobei die Erkrankung oder der Zustand, die/der auf die Modulation des BK-B2-Rezeptors ansprechen, ein Angioödem, wie zum Beispiel ein hereditäres Angioödem, ist.

15. Die feste Dsipersion oder die Zusammensetzung mit verlängerter Freisetzung zur Verwendung nach Anspruch 13 oder 14, wobei die Behandlung eine chronische Behandlung ist.

## Revendications

1. Dispersion solide pour administration orale comprenant:
(a) un antagoniste du récepteur B2 de la bradykinine (BK) ayant une structure chimique selon la formule (1), ou un sel ou solvate de celui-ci: 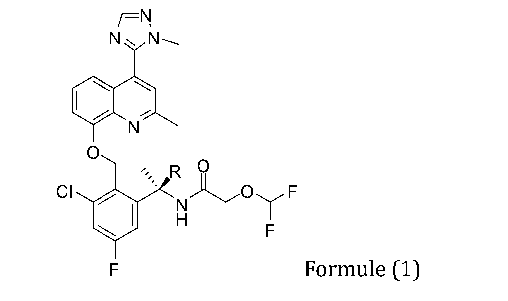 dans laquelle R est un deutérium ou un hydrogène; et
(b) au moins un polymère pharmaceutiquement acceptable;
dans lequel l'antagoniste du récepteur B2 de la BK est amorphe et dispersé de manière homogène dans le polymère.

2. La dsipersion solide selon la revendication 1, dans laquelle l'antagoniste du récepteur B2 de la BK est un composé selon la formule (1) avec R étant le deutérium.

3. La dsipersion solide selon la revendication 1 ou 2, dans laquelle le polymère pharmaceutiquement acceptable est choisi parmi l'hydroxypropylcellulose (HPC), l'hydroxypropylméthylcellulose (HPMC), l'acétate succinate d'hydroxypropylméthylcellulose (HPMCAS), la povidone (PVP), la copovidone (PVP/PA), le polyéthylène glycol (PEG) et l'oxyde de polyéthylène (PEO), et de préférence parmi l'HPMCAS et la copovidone (PVP/PA).

4. La dispersion solide selon l'une quelconque des revendications précédentes, comprenant en outre un tensioactif non ionique choisi dans le groupe des poloxamères et des polysorbates.

5. La dispersion solide selon l'une quelconque des revendications précédentes, dans laquelle la teneur de l'antagoniste du récepteur B2 de la BK est dans la plage de 15 % en poids à 50 % en poids, sur la base du poids total de la dispersion solide.

6. La dispersion solide selon la revendication 5, comprenant
(i) environ 15 % en poids à environ 45 % en poids d'antagoniste du récepteur B2 de la BK de formule (1) et environ 55 % en poids à environ 85 % en poids de HPMCAS, sur la base du poids total de la dispersion solide; ou
(ii) 30 % en poids à 50 % en poids d'antagoniste du récepteur B2 de la BK de formule (1), 50 % en poids à 70 % en poids de copovidone, et éventuellement jusqu'à 5 % en poids de poloxamère, sur la base du poids total de la dispersion solide.

7. La dispersion solide selon l'une quelconque des revendications précédentes, se présentant sous la forme d'une poudre ou de granulés.

8. Procédé de préparation de la dispersion solide selon l'une quelconque des revendications précédentes, comprenant une étape de (i) extrusion à chaud d'un mélange de poudre ou de granulés comprenant l'antagoniste du récepteur B2 de la BK et le polymère pharmaceutiquement acceptable, ou de (ii) séchage par atomisation d'une solution organique de l'antagoniste du récepteur B2 de la BK et du polymère pharmaceutiquement acceptable.

9. Le procédé selon la revendication 8, dans lequel l'étape d'extrusion à chaud est réalisée avec
(i) un mélange de poudre ou de granulés comprenant environ 15 % en poids à environ 45 % en poids d'antagoniste du récepteur B2 de la BK et environ 55 % en poids à environ 85 % en poids de HPMCAS, sur la base du poids total du mélange de poudre ou de granulés; ou
(ii) un mélange de poudre ou de granulés comprenant 30 % en poids à 50 % en poids d'antagoniste du récepteur B2 de la BK et 50 % en poids à 70 % en poids de copovidone, et éventuellement jusqu'à 5 % en poids de poloxamère, sur las base du poids total du mélange de poudre ou de granulés.

10. Utilisation de la dispersion solide selon l'une quelconque des revendications 1 à 7 dans la préparation d'une composition pharmaceutique pour la libération prolongée de l'antagoniste du récepteur B2 de la BK.

11. Composition pharmaceutique à libération prolongée comprenant la dispersion solide selon l'une quelconque des revendications 1 à 7, dans laquelle ladite composition se présentant sous la forme d'un comprimé matriciel, et dans laquelle le comprimé matriciel comprenant 20 % en poids à 40 % en poids de la dispersion solide, sur la base du poids total du comprimé.

12. La composition à libération prolongée selon la revendication 11, dans laquelle le comprimé matriciel comprend en outre un polymère hydrophile formant une matrice et une charge de comprimé, et, éventuellement, un agent glissant et/ou un lubrifiant, et dans laquelle le polymère hydrophile formant une matrice est de l'HPMC, ou une combinaison de deux ou plusieurs qualités d'HPMC.

13. La dispersion solide selon l'une quelconque des revendications 1 à 7, ou la composition à libération prolongée selon la revendication 11 ou 12, destinée à être utilisée dans le traitement d'un sujet souffrant d'une maladie ou d'un état répondant à la modulation du récepteur B2 de la BK, dans laquelle la maladie ou l'état répondant à la modulation du récepteur B2 de la BK est choisi dans le groupe comprenant: un trouble cutané; une maladie oculaire; une maladie de l'oreille; une maladie de la bouche, de la gorge et des voies respiratoires; une maladie gastro-intestinale; une maladie du foie, de la vésicule biliaire et du pancréas; une maladie des voies urinaires et des reins; une maladie des organes génitaux masculins et organes génitaux féminins; une maladie du système hormonal; une maladie métabolique; une maladie cardiovasculaire; une maladie du sang; une maladie lymphatique; un trouble du système nerveux central; un trouble cérébral; une maladie du système musculo-squelettique; un trouble allergique; une douleur; une maladie infectieuse; un trouble inflammatoire; une blessure; un trouble immunologique; cancer; une maladie héréditaire; et œdème.

14. La dsipersion solide ou la composition à libération prolongée destiné à être utilisé selon la revendication 13, dans laquelle la maladie ou état répondant à la modulation du récepteur B2 de la BK est l'angio-œdème, tel que l'angio-œdème héréditaire.

15. La dsipersion solide ou la composition à libération prolongée destiné à être utilisé selon la revendication 13 ou 14, dans laquelle le traitement est un traitement chronique.
